# EUROPEAN PATENT APPLICATION

(11) **EP 3 747 902 A1**
(43) Date of publication of application: **09.12.2020**
(21) Application number: 18903480.4
(22) Date of filing: 30.11.2018
(51) Int. Cl.: C07K 16/10, G01N 33/569

(54) **BINDING MOLECULE HAVING NEUTRALIZING ACTIVITY AGAINST MIDDLE EAST RESPIRATORY SYNDROME-CORONAVIRUS**

(30) Priority: 31.01.2018 KR 20180011776; 11.09.2018 KR 20180108125; 12.11.2018 KR 20180138228
(71) Applicant: Celltrion, Inc., Incheon 22014 (KR)
(72) Inventor: LEE, Soo Young, Incheon 22014 (KR); YI, Kye Sook, Incheon 22014 (KR); KIM, Cheol Min, Incheon 22014 (KR); SONG, Kyung Min, Incheon 22014 (KR); BAE, Yeon Jin, Incheon 22014 (KR); KIM, Woo Joo, Seoul 02841 (KR); CHEONG, Hee Jin, Seoul 02841 (KR); SONG, Joon Young, Seoul 02841 (KR); PARK, Man Seong, Seoul 02841 (KR); NOH, Ji Yun, Seoul 02841 (KR)
(74) Representative: Schlief, Thomas P.
(86) International application number: PCT/KR2018/015141
(87) International publication number: WO 2019/151632

(57) **Abstract**

The present invention relates to a binding molecule having neutralizing activity against Middle East respiratory syndrome-coronavirus (MERS-CoV). More particularly, the present invention relates to a binding molecule having a superior ability to bind to the S protein of MERS-CoV and also having a neutralizing effect on MERS-CoV and is very useful in preventing, treating, or diagnosing MERS-CoV infection.

## Description

### Technical Field

The present invention relates to a binding molecule having neutralizing activity against Middle East Respiratory Syndrome - Coronavirus (MERS-CoV). More particularly, the present invention relates to a binding molecule having strong ability to bind to a spike protein (S protein) on the surface of MERS-CoV and neutralizing activity against MERS-CoV and thus being very useful in the prevention, treatment or diagnosis of MERS-CoV infection.

### Background Art

Middle East Respiratory Syndrome - Coronavirus (MERS-CoV) is an infectious disease caused by coronavirus belonging to *Betacoronavirus,* and the first causative virus was discovered in an unexplained pneumonia patient in the Middle East in 2012. MERS-CoV is believed to have originated from bats and has been known to date to be introduced into humans through camels. Although the propagation pathway thereof has not been fully characterized, it appears that the virus was repeatedly passed from camels to humans in the Middle East, and limited and non-persistent human-to-human transmission occurs.

MERS-CoV has occurred in 27 countries so far, with 2,078 patients from September 2012 to September 29, 2017. Among these patients, 730 died, indicating a fatality rate of 35.1% (WHO). There are no specific therapeutic or preventive agents yet, but the role of antiviral drugs has not been clearly demonstrated. Considering the high mortality and morbidity of MERS-CoV, active treatment using antiviral drugs, such as combination therapy with ribavirin, interferon alpha-2α, and lopinavir/ritonavir, is recommended in early stages of the disease, but is problematic due to side effects thereof.

Meanwhile, as a conventional technique for MERS-CoV-binding antibodies, Korean Patent No. 10-1593641 discloses an antibody that recognizes MERS-CoV nucleocapsid, a diagnostic composition comprising the same, a kit, and a method of detecting MERS-CoV using the same. This document relates to the determination of MERS-CoV infection using an antibody that specifically binds to the nucleocapsid of MERS-CoV, but the neutralizing activity of the antibody against MERS-CoV is unknown, so there is a continuing need for antibodies having a therapeutic effect on MERS-CoV.

### Disclosure

### Technical Problem

Therefore, the present inventors have developed a binding molecule having the ability to bind to an S protein of MERS-CoV in order to solve the problems encountered in the related art, and have ascertained that the binding molecule has neutralizing efficacy against MERS-CoV, thus culminating in the present invention.

An objective of the present invention is to provide a binding molecule that binds to an S protein of MERS-CoV and thus has neutralizing activity against MERS-CoV.

Another objective of the present invention is to provide a composition for preventing or treating MERS-CoV comprising the binding molecule.

Still another objective of the present invention is to provide a kit for diagnosing MERS-CoV comprising the binding molecule.

### Technical Solution

In order to accomplish the above objectives, an embodiment of the present invention provides a neutralizing binding molecule, which binds to a spike protein (S protein) on the surface of MERS-CoV (Middle East Respiratory Syndrome - Coronavirus).

Another embodiment of the present invention provides a composition for preventing or treating MERS-CoV comprising the binding molecule.

Still another embodiment of the present invention provides a kit for diagnosing MERS-CoV comprising the binding molecule.

Hereinafter, a detailed description will be given of the present invention.

An embodiment of the present invention pertains to a neutralizing binding molecule, which binds to an S protein of MERS-CoV.

An embodiment of the present invention pertains to a neutralizing binding molecule, which is any one selected from the group consisting of binding molecules i) to vi) below.
i) a binding molecule comprising a) a heavy-chain variable region comprising a CDR1 region of SEQ ID NO: 1, a CDR2 region of SEQ ID NO: 2, and a CDR3 region of SEQ ID NO: 3, and b) a light-chain variable region comprising a CDR1 region of SEQ ID NO: 4, a CDR2 region of SEQ ID NO: 5, and a CDR3 region of SEQ ID NO: 6
ii) a binding molecule comprising a) a heavy-chain variable region comprising a CDR1 region of SEQ ID NO: 7, a CDR2 region of SEQ ID NO: 8, and a CDR3 region of SEQ ID NO: 9, and b) a light-chain variable region comprising a CDR1 region of SEQ ID NO: 10, a CDR2 region of SEQ ID NO: 11, and a CDR3 region of SEQ ID NO: 12
iii) a binding molecule comprising a) a heavy-chain variable region comprising a CDR1 region of SEQ ID NO: 13, a CDR2 region of SEQ ID NO: 14, and a CDR3 region of SEQ ID NO: 15, and b) a light-chain variable region comprising a CDR1 region of SEQ ID NO: 16, a CDR2 region of SEQ ID NO: 17, and a CDR3 region of SEQ ID NO: 18
iv) a binding molecule comprising a) a heavy-chain variable region comprising a CDR1 region of SEQ ID NO: 19, a CDR2 region of SEQ ID NO: 20, and a CDR3 region of SEQ ID NO: 21, and b) a light-chain variable region comprising a CDR1 region of SEQ ID NO: 22, a CDR2 region of SEQ ID NO: 23, and a CDR3 region of SEQ ID NO: 24
v) a binding molecule comprising a) a heavy-chain variable region comprising a CDR1 region of SEQ ID NO: 25, a CDR2 region of SEQ ID NO: 26, and a CDR3 region of SEQ ID NO: 27, and b) a light-chain variable region comprising a CDR1 region of SEQ ID NO: 28, a CDR2 region of SEQ ID NO: 29, and a CDR3 region of SEQ ID NO: 30
vi) a binding molecule comprising a) a heavy-chain variable region comprising a CDR1 region of SEQ ID NO: 31, a CDR2 region of SEQ ID NO: 32, and a CDR3 region of SEQ ID NO: 33, and b) a light-chain variable region comprising a CDR1 region of SEQ ID NO: 34, a CDR2 region of SEQ ID NO: 35, and a CDR3 region of SEQ ID NO: 36

In an embodiment of the present invention, the binding molecule comprises antibody 1 to antibody 36, as shown in Table 1 below.

**[Table 1]**

| Classification | Heavy-chain CDR1 | Heavy-chain CDR2 | Heavy-chain CDR3 | Light-chain CDR1 | Light-chain CDR2 | Light-chain CDR3 |
|---|---|---|---|---|---|---|
| Antibody 1 | SEQ ID NO: 1 | SEQ ID NO: 2 | SEQ ID NO: 3 | SEQ ID NO: 4 | SEQ ID NO: 5 | SEQ ID NO: 6 |
| Antibody 2 | SEQ ID NO: 7 | SEQ ID NO: 8 | SEQ ID NO: 9 | SEQ ID NO: 10 | SEQ ID NO: 11 | SEQ ID NO: 12 |
| Antibody 3 | SEQ ID NO: 13 | SEQ ID NO: 14 | SEQ ID NO: 15 | SEQ ID NO: 16 | SEQ ID NO: 17 | SEQ ID NO: 18 |
| Antibody 4 | SEQ ID NO: 19 | SEQ ID NO: 20 | SEQ ID NO: 21 | SEQ ID NO: 22 | SEQ ID NO: 23 | SEQ ID NO: 24 |
| Antibody 5 | SEQ ID NO: 25 | SEQ ID NO: 26 | SEQ ID NO: 27 | SEQ ID NO: 28 | SEQ ID NO: 29 | SEQ ID NO: 30 |
| Antibody 6 | SEQ ID NO: 31 | SEQ ID NO: 32 | SEQ ID NO: 33 | SEQ ID NO: 34 | SEQ ID NO: 35 | SEQ ID NO: 36 |

In the present invention, CDRs of variable regions were determined through a typical method using a system devised by Kabat *et al.* (Kabat et al, Sequences of Proteins of Immunological Interest (5th), National Institutes of Health, Bethesda, MD. (1991)). The CDR numbering used in the present invention is determined using the Kabat method, but the present invention also encompasses binding molecules comprising CDRs determined through other methods such as an IMGT method, Chothia method, AbM method and the like.

An embodiment of the present invention pertains to a neutralizing binding molecule, which is any one selected from the group consisting of binding molecules i) to vi) below.
i) a binding molecule comprising a) a heavy-chain variable region having a sequence identity of 95% or more to a heavy-chain variable region of a polypeptide sequence of SEQ ID NO: 37, and b) a light-chain variable region having a sequence identity of 95% or more to a light-chain variable region of a polypeptide sequence of SEQ ID NO: 38
ii) a binding molecule comprising a) a heavy-chain variable region having a sequence identity of 95% or more to a heavy-chain variable region of a polypeptide sequence of SEQ ID NO: 39, and b) a light-chain variable region having a sequence identity of 95% or more to a light-chain variable region of a polypeptide sequence of SEQ ID NO: 40
iii) a binding molecule comprising a) a heavy-chain variable region having a sequence identity of 95% or more to a heavy-chain variable region of a polypeptide sequence of SEQ ID NO: 41, and b) a light-chain variable region having a sequence identity of 95% or more to a light-chain variable region of a polypeptide sequence of SEQ ID NO: 42
iv) a binding molecule comprising a) a heavy-chain variable region having a sequence identity of 95% or more to a heavy-chain variable region of a polypeptide sequence of SEQ ID NO: 43, and b) a light-chain variable region having a sequence identity of 95% or more to a light-chain variable region of a polypeptide sequence of SEQ ID NO: 44
v) a binding molecule comprising a) a heavy-chain variable region having a sequence identity of 95% or more to a heavy-chain variable region of a polypeptide sequence of SEQ ID NO: 45, and b) a light-chain variable region having a sequence identity of 95% or more to a light-chain variable region of a polypeptide sequence of SEQ ID NO: 46
vi) a binding molecule comprising a) a heavy-chain variable region having a sequence identity of 95% or more to a heavy-chain variable region of a polypeptide sequence of SEQ ID NO: 47, and b) a light-chain variable region having a sequence identity of 95% or more to a light-chain variable region of a polypeptide sequence of SEQ ID NO: 48

In an embodiment of the present invention, the binding molecule comprises antibody 1 to antibody 6, as shown in Table 2 below.

**[Table 2]**

| Classificatior | Heavy-chain variable region | Light-chain variable region | Classification | Heavy-chain variable region | Light-chain variable region |
|---|---|---|---|---|---|
| Antibody 1 | SEQ ID NO: 37 | SEQ ID NO: 38 | Antibody 4 | SEQ ID NO: 43 | SEQ ID NO: 44 |
| Antibody 2 | SEQ ID NO: 39 | SEQ ID NO: 40 | Antibody 5 | SEQ ID NO: 45 | SEQ ID NO: 46 |
| Antibody 3 | SEQ ID NO: 41 | SEQ ID NO: 42 | Antibody 6 | SEQ ID NO: 47 | SEQ ID NO: 48 |

In an embodiment of the present invention, the binding molecule may be a Fab fragment, a Fv fragment, a diabody, a chimeric antibody, a humanized antibody, or a human antibody, but is not limited thereto. An embodiment of the present invention provides a fully human antibody that binds to an S protein. As used herein, the term "antibody" is used to have as broad a meaning as possible, and particularly includes an intact monoclonal antibody, a polyclonal antibody, a multispecific antibody formed from two or more intact antibodies (e.g. a bispecific antibody), and an antibody fragment that shows the desired biological activity. The antibody is a protein that is produced by an immune system capable of recognizing a specific antigen and binding thereto. The antibody is typically configured to have a Y-shaped protein comprising four amino acid chains (two heavy chains and two light chains). Each antibody has two regions including a variable region and a constant region. The variable region, which is located at the ends of the arms of a Y, binds to the target antigen and interacts therewith. The variable region includes a complementarity-determining region (CDR) that recognizes the specific binding site on the specific antigen and binds thereto. The constant region, which is located at the tail of the Y, is recognized by the immune system and interacts therewith. The target antigen has a plurality of binding sites called epitopes, recognized by CDRs on antibodies. Respective antibodies specifically binding to different epitopes have different structures. Therefore, a single antigen may have at least one antibody corresponding thereto.

Moreover, the present invention includes a functional variant of the binding molecule. Such binding molecules are regarded as functional variants of the binding molecule of the present invention so long as the variants are capable of competing with the binding molecule of the present invention in order to specifically bind to MERS-CoV or to an S protein thereof and also have neutralizing activity against MERS-CoV. Such functional variants include, but are not limited to, derivatives, the primary conformational sequences of which are substantially similar, and examples thereof include *in-vitro* or *in-vivo* modifications, chemicals and/or biochemicals, and they are not found in the parent monoclonal antibody of the present invention. Examples of such modifications may include acetylation, acylation, covalent bonding of nucleotides or nucleotide derivatives, covalent bonding of lipids or lipid derivatives, crosslinking, disulfide bonding, glycosylation, hydroxylation, methylation, oxidation, pegylation, proteolysis and phosphorylation. The functional variant may selectively be an antibody comprising an amino acid sequence resulting from subjecting at least one amino acid to substitution, insertion, deletion or combinations thereof, compared to the amino acid sequence of the parent antibody. Furthermore, the functional variant may include a truncated form of the amino acid sequence in one or both of an amino terminus and a carboxyl terminus. The functional variant of the present invention may have binding affinity the same as or different from, i.e. higher or lower than, that of the parent antibody of the present invention, but may still bind to MERS-CoV or to an S protein thereof. For example, the amino acid sequence of the variable region, including, but not limited to, a framework structure or a hypervariable region, especially a CDR (complementarity-determining region) of a light chain or heavy chain, may be modified. Typically, a light-chain or heavy-chain region includes three hypervariable regions comprising three CDRs and more conserved regions, namely framework regions (FRs). The hypervariable region includes an amino acid residue from a CDR and an amino acid residue from a hypervariable loop. A functional variant that falls within the scope of the present invention may have an amino acid sequence homology of about 50% to 99%, about 60% to 99%, about 80% to 99%, about 90% to 99%, about 95% to 99%, or about 97% to 99% with the parent antibody of the present invention. In order to optimally arrange amino acid sequences to be compared, and also, in order to define similar or identical amino acid residues, among computer algorithms, Gap or Best-fit, known to those skilled in the art, may be used. The functional variant may be obtained by subjecting the parent antibody or a portion thereof to a known molecular biological process including PCR or mutagenesis/partial mutagenesis using an oligomer nucleotide, or to an organic synthesis process, but the present invention is not limited thereto.

Also, a drug may be additionally attached to the binding molecule. Specifically, the binding molecule according to the present invention may be used in the form of an antibody-drug conjugate containing the drug conjugated thereto. When the antibody-drug conjugate (ADC), that is, the immunoconjugate, is used to topically deliver the drug, targeted delivery of the drug moiety to infected cells becomes possible. When the drug agent is administered without being conjugated, unacceptable levels of toxicity to normal cells may be caused. By increasing not only the drug conjugation and the drug releasability but also the selectivity of the polyclonal antibody and the monoclonal antibody (mAb), maximum efficacy and minimum toxicity of ADC may be obtained.

The use of typical means for attaching the drug moiety to the antibody, for example, covalent bonding, may cause the production of heterogeneous molecular mixtures in which the drug moiety is attached to many sites on the antibody. For example, a cytotoxic drug is conjugated to the antibody through many lysine residues of the antibody to thus produce a heterogeneous antibody-drug conjugate mixture. Depending on the reaction conditions, such a heterogeneous mixture typically has a distribution whereby the number of antibodies attached to the drug moiety ranges from 0 to about 8 or more. Furthermore, each subgroup of the conjugate comprising the drug moiety and the antibody at a specific integer ratio is a potential heterogeneous mixture in which the drug moiety is attached to various sites on the antibody. Antibodies are biomolecules that are large, complicated and structurally various, and often have many reactive functional groups. The reactivity of a linker reagent and a drug-linker intermediate is dependent on factors such as pH, concentration, salt concentration, and cosolvents.

In addition, an embodiment of the present invention provides a nucleic acid molecule encoding the binding molecule.

The nucleic acid molecule of the present invention includes any nucleic acid molecule in which the amino acid sequence of the antibody provided in the present invention is translated into a polynucleotide sequence as known to those skilled in the art. Thus, various polynucleotide sequences may be prepared using an ORF (open reading frame), and may also be incorporated in the nucleic acid molecule of the present invention.

In addition, an embodiment of the present invention provides an expression vector into which the nucleic acid molecule is inserted.

The expression vector may include, but is not limited to, any one selected from the group consisting of an expression vector available from Celltrion, such as a MarEx vector (Korean Patent No. 10-1076602), and a commercially widely useful pCDNA vector, F, R1, RP1, Co1, pBR322, ToL, and Ti vector; a cosmid; phages, such as lambda, lambdoid, M13, Mu, p1 P22, Qµ, T-even, T2, T3, T7, etc.; and plant viruses, and any expression vector known to those skilled in the art may be used in the present invention, and the expression vector may be selected depending on the properties of the host cell of interest. The introduction of the vector into the host cell may be performed through calcium phosphate transfection, viral infection, DEAE-dextran-mediated transfection, lipofectamine transfection, or electroporation, but the present invention is not limited thereto, and those skilled in the art may adopt an introduction process suitable for the expression vector and the host cell. For example, the expression vector may contain at least one selection marker, but is not limited thereto, and selection is possible depending on whether or not the product is capable of being obtained using the vector not containing the selection marker. Choosing the selection marker depends on the host cell of interest, and is performed using any process known to those skilled in the art, and thus the present invention is not limited in connection therewith.

In order to easily purify the binding molecule of the present invention, a tag sequence may be inserted into the expression vector and thus fused therewith. The tag may include, but is not limited to, a hexa-histidine tag, a hemagglutinin tag, a myc tag or a flag tag, and any tag may be useful in the present invention so long as it facilitates purification as known to those skilled in the art.

In addition, an embodiment of the present invention provides a cell line in which the expression vector is transformed into a host cell to produce the binding molecule that binds to MERS-CoV and thus has neutralizing activity.

In the present invention, the cell line may include, but is not limited to, mammals, plants, insects, fungi, or cells of cellular origin. Any one selected from the group consisting of mammalian cells, such as CHO cells, F2N cells, COS cells, BHK cells, Bowes melanoma cells, HeLa cells, 911 cells, HT1080 cells, A549 cells, HEK 293 cells and HEK293T cells may be used as the host cell, but the present invention is not limited thereto, and any cells may be used, so long as they are useful as host cell for mammals, as known to those skilled in the art.

In addition, an embodiment of the present invention pertains to a composition for the prevention or treatment of MERS-CoV infection comprising the binding molecule. The composition of the present invention may include a pharmaceutically acceptable excipient, in addition to the binding molecule. Such pharmaceutically acceptable excipients are well known to those skilled in the art.

The composition of the present invention may further include at least one other therapeutic agent or diagnostic agent. For example, the composition of the present invention may further include, as an antiviral drug, interferon, an anti-S protein monoclonal antibody, an anti-S protein polyclonal antibody, a nucleoside analogue, a DNA polymerase inhibitor, a siRNA preparation or a therapeutic vaccine, in addition to the binding molecule.

The composition of the present invention comprising the binding molecule may be provided in the form of a formulation, such as a sterile injectable solution, a lyophilized formulation, a pre-filled syringe solution, an oral formulation, a formulation for external use or a suppository through respective typical processes, but the present invention is not limited thereto.

Also, the composition of the present invention comprising the binding molecule may be administered in an oral or parenteral manner. For example, the administration route may be intravenous administration, but is not limited thereto.

The composition of the present invention is administered to a mammal including a human, thereby preventing or treating MERS-CoV infection and diseases caused by MERS-CoV infection. Here, the amount of the binding molecule (e.g. antibody) that is administered depends on the treatment subject, severity of disease or status, administration rate and doctor's prescription.

In addition, an embodiment of the present invention pertains to a diagnostic kit comprising the binding molecule. The binding molecule of the present invention used in the diagnostic kit may be detectably labeled. Various methods that may be used to label biomolecules are well known to those skilled in the art, and are considered to fall within the scope of the present invention. Examples of labels useful in the present invention may include enzymes, radioisotopes, colloidal metals, fluorescent compounds, chemiluminescent compounds and bioluminescent compounds. Commonly used labels include fluorescent substances (e.g., fluorescein, rhodamine, Texas red, etc.), enzymes (such as horseradish peroxidase, β-galactosidase, or alkaline phosphatase), radioisotopes (e.g. 32P or 1251), biotin, digoxigenin, colloidal metals, or chemiluminescent or bioluminescent compounds (such as dioxetane, luminol or acridinium). Labeling methods such as covalent bonding, iodination, phosphorylation, biotinylation, etc. of enzymes or biotinyl groups are well known in the art. Detection methods include, but are not limited to, autoradiography, fluorescence microscopy, direct and indirect enzyme reactions, and the like. A commonly used detection assay is the radioisotope or non-radioisotope method. Particularly useful are western blotting, overlay analysis, RIA (radioimmunoassay), IRMA (immunoradioimmunometric assay), EIA (enzyme immunoassay), ELISA (enzyme-linked immunosorbent assay), FIA (fluorescent immunoassay) and CLIA (chemiluminescent immunoassay).

The diagnostic kit of the present invention may be used to detect the presence or absence of MERS-CoV by contacting a sample with the binding molecule and observing the reaction.

The sample may be, but is not limited to, any one selected from the group consisting of sputum, saliva, blood, sweat, lung cells, mucus of lung tissue, respiratory tissue and spit of a subject, and the sample may be prepared using a process typically known to those skilled in the art.

In addition, an embodiment of the present invention provides a kit for the diagnosis, prevention or treatment of a disease caused by MERS-CoV, comprising:
a) the binding molecule; and
b) a vessel.

In the kit for diagnosis, prevention or treatment according to the present invention, a solid carrier may be included in the vessel thereof. The antibody of the present invention may be attached to the solid carrier, and the solid carrier may be porous or non-porous, or may be planar or non-planar.

In addition, the present invention provides a method of diagnosing, preventing or treating a disease caused by MERS-CoV infection comprising administering the above composition in a therapeutically effective amount to a subject having a disease caused by MERS-CoV infection.

In an embodiment of the present invention, the diagnosis, prevention or treatment method may further include administering an antiviral drug, a virus entry inhibitor or a virus adhesion inhibitor.

The terms used in the present invention are defined as follows.

As used herein, the term "binding molecule" refers to an intact immunoglobulin including monoclonal antibodies, such as chimeric, humanized or human monoclonal antibodies, or to an antigen-binding fragment, which is an immunoglobulin that binds to an antigen. For example, it indicates a variable region, enzyme, receptor or protein which comprises an immunoglobulin fragment that competes with the intact immunoglobulin in order to bind to a spike protein of MERS-CoV. Regardless of the structure, an antigen-binding fragment binds with the same antigen that is recognized by the intact immunoglobulin. The antigen-binding fragment may comprise a peptide or polypeptide comprising an antibody amino acid sequence consisting of 2 or more contiguous amino acid residues, 20 or more contiguous amino acid residues, 25 or more contiguous amino acid residues, 30 or more contiguous amino acid residues, 35 or more contiguous amino acid residues, 40 or more contiguous amino acid residues, 50 or more contiguous amino acid residues, 60 or more contiguous amino acid residues, 70 or more contiguous amino acid residues, 80 or more contiguous amino acid residues, 90 or more contiguous amino acid residues, 100 or more contiguous amino acid residues, 125 or more contiguous amino acid residues, 150 or more contiguous amino acid residues, 175 or more contiguous amino acid residues, 200 or more contiguous amino acid residues, or 250 or more contiguous amino acid residues.

As used herein, the term "antigen-binding fragment" indicates Fab, F(ab'), F(ab')2, Fv, dAb, Fd, complementarity-determining region (CDR) fragments, single-chain antibodies (scFv), bivalent single-chain antibodies, single-chain phage antibodies, diabodies, triabodies, tetrabodies, polypeptides that contain at least one fragment of an immunoglobulin that is sufficient to confer specific antigen binding to the polypeptide, etc. The above fragments may be produced synthetically or through enzymatic or chemical cleavage of intact immunoglobulins, or they may be genetically engineered through recombinant DNA techniques. Such production methods are well known in the art.

As used herein, the term "pharmaceutically acceptable excipient" means any inert substance that is combined with an active molecule such as a drug, agent, or antibody for preparing an acceptable or convenient dosage form. The pharmaceutically acceptable excipient is an excipient that is non-toxic or at least of reduced toxicity to recipients at typical usage dosages and concentrations, and is compatible with other ingredients of the formulation comprising the drug, agent or binding molecule.

As used herein, the term "therapeutically effective amount" refers to an amount of the binding molecule of the present invention that is effective for prevention or treatment before or after exposure to MERS-CoV.

### Advantageous Effects

According to the present invention, the binding molecule has strong ability to bind to an S protein of MERS-CoV and thereby exhibits neutralizing activity and is thus very useful in the prevention, treatment or diagnosis of MERS-CoV infection.

### Brief Description of Drawings

FIG. 1 shows the neutralizing activity depending on the antibody concentration against Korean isolate MERS-CoV (MERS-CoV/Korea/KNIH/002_05_2015) by performing a plaque assay with two finally selected antibodies;
FIG. 2 shows the virus titer through a plaque assay after tissue culture by infecting human lung tissue with Korean isolate MERS-CoV and an antibody in order to evaluate the neutralizing activity of the antibody using a human lung tissue infection model (*ex vivo*);
FIG. 3A shows the results of quantitative PCR for evaluation of animal treatment efficacy using an animal model capable of MERS-CoV infection and proliferation (hDPP4 (human dipeptidyl peptidase 4) receptor-overexpressing mouse) and an antibody binding to the virus;
FIG. 3B shows the results of a plaque assay for evaluation of animal treatment efficacy using an animal model capable of MERS-CoV infection and proliferation (hDPP4 (human dipeptidyl peptidase 4) receptor-overexpressing mouse) and an antibody binding to the virus;
FIG. 4A shows the results of quantitative PCR for evaluation of preventive efficacy of an antibody against MERS-CoV using an animal model capable of MERS-CoV infection and proliferation (hDPP4 (human dipeptidyl peptidase 4) receptor-overexpressing mouse) (*p<0.05, **p<0.01, ***p<0.001);
FIG. 4B shows the results of a plaque assay for evaluation of preventive efficacy of an antibody against MERS-CoV using an animal model capable of MERS-CoV infection and proliferation (hDPP4 (human dipeptidyl peptidase 4) receptor-overexpressing mouse) (*p<0.05, **p<0.01, ***p<0.001);
FIG. 5 shows histological changes in mouse lung for evaluation of preventive efficacy of an antibody against MERS-CoV using an animal model capable of MERS-CoV infection and proliferation (hDPP4 (human dipeptidyl peptidase 4) receptor-overexpressing mouse);
FIG. 6A shows the mouse body weight reduction for evaluation of therapeutic efficacy of an antibody against MERS-CoV using an animal model capable of MERS-CoV infection and proliferation (hDPP4 (human dipeptidyl peptidase 4) receptor-overexpressing mouse) (*p<0.05, **p<0.01, ***p<0.001);
FIG. 6B shows the mouse survival rate for evaluation of therapeutic efficacy of an antibody against MERS-CoV using an animal model capable of MERS-CoV infection and proliferation (hDPP4 (human dipeptidyl peptidase 4) receptor-overexpressing mouse) (*p<0.05, **p<0.01, ***p<0.001);
FIG. 6C shows the results of quantitative PCR for evaluation of therapeutic efficacy of an antibody against MERS-CoV using an animal model capable of MERS-CoV infection and proliferation (hDPP4 (human dipeptidyl peptidase 4) receptor-overexpressing mouse) (*p<0.05, **p<0.01, ***p<0.001); and
FIG. 6D shows the results of a plaque assay for evaluation of therapeutic efficacy of an antibody against MERS-CoV using an animal model capable of MERS-CoV infection and proliferation (hDPP4 (human dipeptidyl peptidase 4) receptor-overexpressing mouse) (*p<0.05, **p<0.01, ***p<0.001).

### Mode for Invention

A better understanding of the present invention may be obtained via the following examples, which are set forth to illustrate and are not to be construed as limiting the scope of the present invention. The documents cited herein are incorporated by reference into this application.

### Example 1: Isolation of PBMCs from blood of patients recovered from MERS-CoV

Blood donors were those who were confirmed to have been infected with MERS-CoV in 2015 and no longer exhibited viruses as a result of treatment, and the donor selection and blood collection processes were performed under the approval of the Institutional Review Board (IRB). After donor selection, about 30 ml of whole blood was collected, and PBMCs (peripheral blood mononuclear cells) were isolated using a Ficoll-Paque™ PLUS (GE Healthcare) method. The isolated PBMCs were washed two times with a phosphate buffer solution and then stored in a liquid nitrogen tank at a concentration of 1x10⁷ cells/ml in a freezing medium (RPMI:FBS:DMSO = 5:4:1).

### Example 2: Production of antibody-displayed phage library

Total RNA was extracted from the PBMCs isolated in Example 1 using a TRIzol reagent (Invitrogen), after which cDNA was synthesized using a SuperScript™ III First-Strand cDNA synthesis system (Invitrogen, USA).

Production of the antibody library from the synthesized cDNA was performed with reference to the related literature (Barbas C. et. al. Phage Display: A Laboratory Manual. 2001. CSHL Press). Briefly, light-chain and heavy-chain variable regions of the antibody were amplified from the synthesized cDNA through a PCR (polymerase chain reaction) method using high-fidelity Taq polymerase (Roche) and a degenerative primer set (IDT). The isolated light-chain and heavy-chain variable-region fragments were made into a scFv gene through an overlap PCR method so as to be connected as one sequence in random combination, followed by amplification, cleavage with a restriction enzyme, and isolation of scFv using 1% agarose gel electrophoresis and a gel extraction kit (Qiagen). A phage vector was cleaved with the same restriction enzyme, isolated, mixed with the scFv gene, added with T4 DNA ligase (New England Biolabs), and then allowed to react at 16°C for 12 hr or more. The resulting reaction solution was mixed with ER2738 competent cells, and was then transformed through an electroporation process. The transformed ER2738 was subjected to shaking culture, added with a VCSM13 helper phage (Agilent Technologies) and cultured for 12 hr or more.

### Example 3: Selection using phage enzyme immunoassay

The phage library culture solution prepared in Example 2 was centrifuged to thus remove host cells, added with 4% PEG and 0.5 M NaCl, and centrifuged, so the phage was precipitated and the supernatant was removed. The precipitated phage was diluted with 1% BSA/TBS to afford a phage library, after which panning was independently performed through binding to and dissociation from various MERS-CoV spike proteins (S proteins), thereby isolating an scFv-phage having the ability to bind to MERS-CoV S protein. For example, the phage library was added to an ELISA plate to which an RBD region (residues 367 to 588 on S1 glycoprotein), which is a portion of the MERS-CoV S protein, was attached, followed by reaction at room temperature for 2 hr. The reaction solution was removed, after which the ELISA plate was washed with PBS containing 0.05% Tween 20 and then added with 60 µℓ of 0.1 M glycine-HCl (pH 2.2), so the scFv-phage was detached from the antigen, and neutralized using 2M Tris (pH 9.1). The scFv-phage thus neutralized was infected with ER2738, cultured with a helper phage and used for subsequent panning. A portion of the infected ER2738 was spread on an LB plate before the addition of the helper phage, and a colony was obtained the next day.

A total of 1,200 colonies, formed each time panning was performed, were added to a culture medium in a 96-well deep well plate (Axygen), subjected to shaking culture, and added with a helper phage when OD₆₀₀ reached 0.7 or more, followed by shaking culture at 37°C for 12 hr or more. The culture solution was centrifuged, so the host cells were removed and the supernatant containing the scFv-phage was prepared.

The scFv-phage supernatant thus prepared was diluted at 1:1 with 6% BSA/PBS, placed in each well of a 96-well microtiter plate to which MERS-CoV S proteins were adsorbed and then blocked, and allowed to stand at 37°C for 2 hr. Each well was washed three times with PBS containing 0.05% Tween 20, added with an anti-M13 antibody labeled with HRP (horseradish peroxidase), and allowed to stand at 37°C for 1 hr. Each well was washed three times with PBS containing 0.05% Tween 20 and then added with ABTS (2,2'-azinobis[3-ethylbenzothiazoline-6-sulfonic acid]-diammonium salt), after which absorbance was measured at 405 nm, whereby 444 scFv-phages having the ability to bind to MERS-CoV S proteins were selected.

### Example 4: Evaluation of binding capacity of antibody fragment (scFv-Fc)

For the 444 scFv-phages selected in Example 3, DNA was obtained through shaking culture of colonies, after which sequences for antibody variable regions were analyzed. Among these, 118 scFv-phages, selected by excluding clones with overlapping amino acid sequences, were cloned into a vector containing an Fc region and converted in the form of an antibody fragment (scFv-Fc) in order to evaluate the expression capacity and neutralizing activity in the candidate antibody animal cell lines. After transfection and expression in F2N cells (Korean Patent No. 10-1005967, Patentee: Celltrion Inc.) using a transfection reagent, the culture solution thereof was used, and the ability of the antibody fragment (scFv-Fc) to bind to three S proteins of MERS-CoV was measured through ELISA. Briefly, MERS-CoV S proteins were attached to the ELISA plate and the expressed antibody fragments were added thereto. After washing the unbound antibody with PBS containing 0.05% Tween 20, antibody fragments bound to the antigen were selected using an anti-human Fc antibody linked with HRP (horseradish peroxidase). Therefore, it was confirmed that 111 antibody fragments specifically bound to S proteins of MERS-CoV.

### Example 5: Evaluation of neutralizing activity of antibody fragment against MERS-CoV (first in-vitro neutralizing activity evaluation)

### 1) Measurement of neutralizing activity against Saudi isolate virus

The 111 antibody fragment culture solutions expressed through the method of Example 4 were sequentially diluted 2-fold to thus prepare samples of 12 concentrations. For Saudi isolate virus (MERS/HCoV/KSA/EMC/2012), a virus stock was dissolved, diluted to a concentration of 25 of TCID₅₀/well, mixed with each of the antibody fragment culture solutions in the 12 concentrations prepared above, allowed to stand at 37°C for 2 hr, transferred to a 96-well plate containing cultured Vero cells, and allowed to stand at 37°C for 1 hr to thus induce infection. The mixed solution of the virus and the antibody fragment culture solution was removed, a culture medium was placed in each well, and culture was performed in a 5% CO₂ incubator at 37°C for 3 days, after which a cytopathic effect in each well was observed using a microscope, and virus-neutralizing activity (the antibody concentration of the well at which 50% neutralizing activity was observed) was calculated (Reed & Muench method). The amount of the virus used per well (25 of TCID₅₀/well) was determined through back calculation. The lower the antibody concentration showing the 50% virus-neutralizing effect, the better the neutralizing activity.

As shown in Table 3 below, it was confirmed that 36 antibody fragments had neutralizing activity superior to positive control antibodies 1 and 2 (binding to MERS-CoV S protein and having known neutralizing effect).

**[Table 3]**

| Classification | 50% Virus neutralization concentration (µg/ml) |
|---|---|
| Antibody 1 | 0.022 |
| Antibody 2 | <=0.01 |
| Antibody 3 | 0.014 |
| Antibody 4 | 0.010 |
| Antibody 5 | 0.003 |
| Antibody 6 | 0.021 |
| Antibody 7 | 0.014 |
| Antibody 8 | 0.056 |
| Antibody 9 | 0.022 |
| Antibody 10 | 0.061 |
| Antibody 11 | 0.034 |
| Antibody 12 | 0.051 |
| Antibody 13 | 0.050 |
| Antibody 14 | 0.011 |
| Antibody 15 | 0.086 |
| Antibody 16 | 0.016 |
| Antibody 17 | 0.047 |
| Antibody 18 | 0.011 |
| Antibody 19 | 0.021 |
| Antibody 20 | 0.023 |
| Antibody 21 | 0.024 |
| Antibody 22 | 0.053 |
| Antibody 23 | 0.019 |
| Antibody 24 | 0.067 |
| Antibody 25 | 0.031 |
| Antibody 26 | 0.011 |
| Antibody 27 | 0.020 |
| Antibody 28 | 0.035 |
| Antibody 29 | 0.048 |
| Antibody 30 | 0.007 |
| Antibody 31 | 0.021 |
| Antibody 32 | 0.060 |
| Antibody 33 | 0.010 |
| Antibody 34 | 0.061 |
| Antibody 35 | 0.033 |
| Antibody 36 | 0.031 |
| Positive control antibody 1 | 0.09 |
| Positive control antibody 2 | 1.69 |

### 2) Measurement of neutralizing activity against Korean isolate virus

The 36 antibody fragments having neutralizing activity against Saudi virus confirmed in 1) above were subjected to a PRNT (plaque reduction neutralization test) with Korean isolate virus (MERS-CoV/Korea/KNIH/002_05_2015).

For PRNT, the antibody sample was diluted, mixed with 100 PFU virus in equal amounts, allowed to react at 37°C for 1 hr, used to infect a cell line, and subjected to a plaque assay. After culture in a 5% CO₂ incubator at 37°C for 3 days and then staining using crystal violet, the number of formed plaques was comparatively analyzed, and the neutralizing activity of the antibody sample was evaluated.

Based on the results of comparative analysis, it was confirmed that the selected antibody fragments reduced plaque formation compared to two positive control antibodies, indicating that the 36 antibody fragments of the present invention had neutralizing activity superior to the two positive control antibodies (the results are not shown).

### Example 6. Evaluation of antibody expression rate and antibody binding specificity after conversion into fully human antibody

The selected antibody fragments were converted into fully human antibodies using the genetic information thereof, an antibody culture solution was prepared through the method of Example 4, and the antigen-binding sites, antibody expression rates, etc. in the fully human antibodies were confirmed. Based on comprehensive results including the above results of evaluation of virus-neutralizing activity, 18 fully human antibodies were selected out of 36.

### Example 7. Evaluation of neutralizing activity of fully human antibody against MERS-CoV (second in-vitro neutralizing activity evaluation)

### 1) Evaluation of neutralizing activity against Saudi isolate virus

The 18 antibodies selected in Example 6 were converted into fully human antibodies, after which the virus-neutralizing activity thereof against Saudi isolate virus was evaluated in the same manner as in 1) of Example 5. As shown in Table 4 below, it was confirmed that 16 of the 18 antibodies had virus-neutralizing activity superior to positive control antibody 1 (binding to MERS-CoV S protein and having known neutralizing effect).

**[Table 4]**

| Classification | 50% Virus neutralization concentration (µg/ml) (Virus back titration 36.1 TCID₅₀/well) | 50% Virus neutralization concentration (µg/ml) (Virus back titration 114.8 TCID₅₀/well) |
|---|---|---|
| Antibody 1 | 0.08 | 0.16 |
| Antibody 2 | 0.07 | 0.07 |
| Antibody 3 | 0.07 | 0.38 |
| Antibody 4 | 0.08 | 0.12 |
| Antibody 5 | 0.02 | 0.04 |
| Antibody 6 | 0.42 | ND |
| Antibody 7 | 0.06 | 0.13 |
| Antibody 9 | 0.08 | 0.18 |
| Antibody 14 | 0.15 | 0.25 |
| Antibody 18 | 0.19 | 0.20 |
| Antibody 21 | 0.11 | 0.16 |
| Antibody 26 | 0.08 | 0.11 |
| Antibody 27 | 0.24 | 0.31 |
| Antibody 29 | 0.39 | 0.78 |
| Antibody 33 | 2.43 | ND |
| Antibody 34 | 0.38 | ND |
| Antibody 35 | 0.11 | 0.20 |
| Antibody 36 | ND | ND |
| Positive control antibody 1 | 1.75 | 2.94 |

### 2) Evaluation of neutralizing activity of fully human antibody against Korean isolate virus

The 18 antibodies selected in Example 6 were converted into fully human antibodies, after which the virus-neutralizing activity thereof against Korean isolate virus was evaluated in the same manner as in 2) of Example 5.

Based on the results of PRNT, as shown in Table 5 below regarding 18 antibodies upon first evaluation (7 antibodies) and upon second evaluation (11 antibodies), it was confirmed that all of the 18 antibodies exhibited low numerical values and thus superior virus-neutralizing activity compared to positive control antibodies 1 and 2 (binding to MERS-CoV S protein and having known neutralizing effect).

**[Table 5]**

| Classification | IC₅₀ (µg/ml) |
|---|---|
| First experiment | |
| Positive control antibody 1 | 0.03875 |
| Positive control antibody 2 | 0.1215 |
| Antibody 1 | 0.0004 |
| Antibody 2 | 0.0009 |
| Antibody 7 | 0.00065 |
| Antibody 9 | 0.0011 |
| Antibody 14 | 0.0015 |
| Antibody 18 | 0.0076 |
| Antibody 21 | 0.00095 |

| Second experiment | |
|---|---|
| Positive control antibody 1 | 0.1038 |
| Positive control antibody 2 | 0.5195 |
| Antibody 3 | 0.0032 |
| Antibody 4 | 0.00165 |
| Antibody 5 | 0.00025 |
| Antibody 6 | 0.0008 |
| Antibody 26 | 0.0039 |
| Antibody 27 | 0.0018 |
| Antibody 29 | 0.00375 |
| Antibody 33 | 0.0232 |
| Antibody 34 | 0.0006 |
| Antibody 35 | 0.00275 |
| Antibody 36 | 0.03325 |

### Example 8. Selection of clone for cell line development

In order to select an antibody for cell line development, property evaluation was further performed in addition to the evaluation of neutralizing activity. The property evaluation was performed for antibody target site, antibody expression rate, and heat resistance. In order to evaluate heat resistance, briefly, an antibody and Sypro Orange (Thermo Fisher Scientific) were diluted to appropriate concentrations and mixed, after which the prepared sample was placed in a PCR 96-well plate, and the fluorescence value was measured by setting a melt curve from 25°C to 99°C using a 7500 Real-Time PCR System (Thermo Fisher Scientific). Based on the comprehensive results of the above neutralizing activity evaluation and the property evaluation shown in Table 6 below, the 6 antibodies shown in Table 5 were selected and cloned into a vector suitable for cell line development.

**[Table 6]**

| Classification | Antibody target site | Antibody expression rate (µg/ml) | Heat resistance (°C) |
|---|---|---|---|
| Antibody 1 | MERS-RBD | 3.142 | 53 |
| Antibody 2 | MERS-RBD | 6.16 | 59 |
| Antibody 3 | MERS-RBD | 4.296 | 57 |
| Antibody 4 | MERS-RBD | 3.687 | 55 |
| Antibody 5 | MERS-RBD | 3.558 | 57 |
| Antibody 6 | MERS-Other | 4.014 | 57 |
| Antibody 7 | MERS-RBD | 2.556 | N/A |
| Antibody 9 | MERS-RBD | 2.088 | N/A |
| Antibody 14 | MERS-RBD | 5.611 | N/A |
| Antibody 18 | MERS-RBD | 3.6 | 55 |
| Antibody 21 | MERS-RBD | 2.494 | 55 |
| Antibody 26 | MERS-RBD | 3.753 | 53 |
| Antibody 27 | MERS-RBD | 6.894 | 57 |
| Antibody 29 | MERS-RBD | 3.897 | N/A |
| Antibody 33 | MERS-S2 | 6.894 | N/A |
| Antibody 34 | MERS-Other | 3.897 | 53 |
| Antibody 35 | MERS-RBD | 4.014 | N/A |
| Antibody 36 | MERS-S2 | 3.558 | 51 |

### Example 9. Evaluation of neutralizing activity against Saudi and Jordan virus (third in-vitro neutralizing activity evaluation)

For the 6 antibodies selected in Example 8, the neutralizing activity thereof against Saudi isolate virus and Jordan isolate virus (MERS-HCoV/Jordan/01) was evaluated in the same manner as in 1) of Example 5. As shown in Table 7 below, the virus-neutralizing activity thereof was superior to four positive control antibodies (binding to MERS-CoV S protein and having known neutralizing effect).

**[Table 7]**

| Classification | Neutralizing activity against Saudi virus (MERS-CoV_KSA/EMC/2012) IC₅₀ (µg/ml) | Neutralizing activity against Jordan virus (MERS-HCoV/Jordan/01) IC₅₀ (µg/ml) |
|---|---|---|
| Antibody 1 | 0.44 | 0.11 |
| Antibody 2 | 0.09 | 0.06 |
| Antibody 3 | 0.13 | 0.11 |
| Antibody 4 | 0.06 | 0.05 |
| Antibody 5 | 0.06 | 0.09 |
| Antibody 6 | 1.11 | 1.54 |
| Positive control antibody 1 | 0.76 | 0.43 |
| Positive control antibody 2 | N/D | N/D |
| Positive control antibody 3 | 0.26 | 0.13 |
| Positive control antibody 4 | 0.22 | 0.26 |

### Example 10. Evaluation of neutralizing activity against Korean isolate virus (third in-vitro neutralizing activity evaluation)

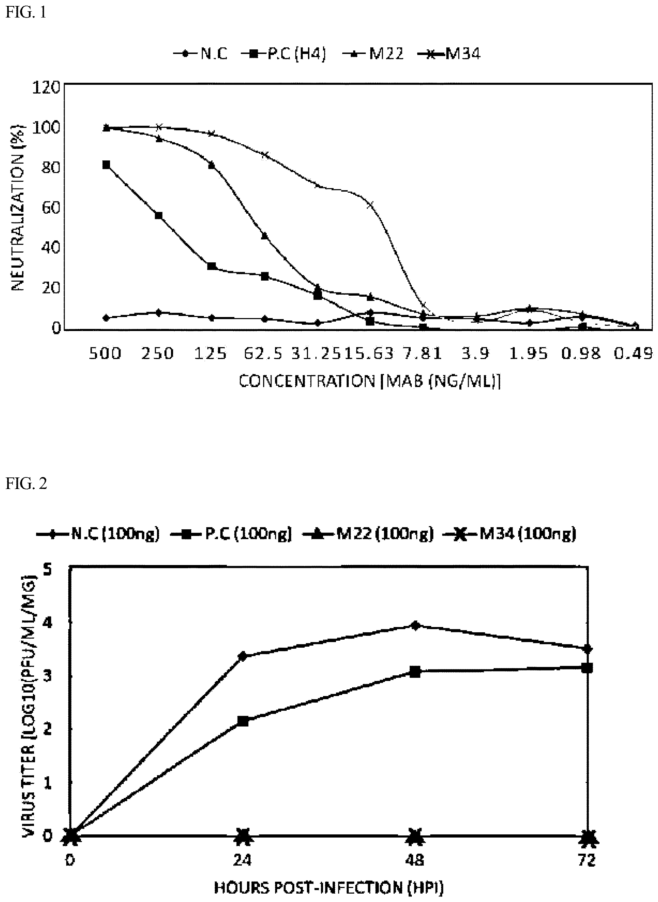

For the two antibodies selected in Example 9, a plaque assay was performed in the same manner as in 2) of Example 5, and the neutralizing activity of the antibodies at different concentrations against Korean isolate MERS-CoV (MERS-CoV/Korea/KNIH/002_05_2015) was evaluated. Therefore, it was confirmed that the two antibodies had superior virus-neutralizing activity compared to positive control antibody 4 (binding to MERS-CoV S protein and having known neutralizing effect) (FIG. 1).

### Example 11. Evaluation of virus-neutralizing activity (ex vivo)

The neutralizing activity of an antibody for cell line development was evaluated using a human lung tissue infection model (*ex vivo*) (Table 8). Briefly, Korean isolate MERS-CoV (MERS-CoV/Korea/KMH/002_05_2015) and an antibody sample for evaluation were prepared based on the IC₅₀ values and allowed to react for 1 hr, after which human lung tissue was infected with the reaction product of the virus and the antibody and tissue culture was carried out. Then, culture was performed for 3 days, during which the culture supernatant was collected at intervals of 24 hr, and the virus titer was measured through a plaque assay. When 100 ng of each of the two antibodies having the efficacy confirmed in Example 10 was allowed to react with MERS-CoV and human lung tissue was then infected therewith, as shown in Table 8 below, it was confirmed that the growth of MERS-CoV was inhibited in the supernatant at 24, 48, and 72 hr compared to negative and positive control antibodies not treated with the antibody (FIG. 2).

**[Table 8]**

| Classification | Lung weight (mg) | Virus content (log10PFU/ml/ng) | | |
|---|---|---|---|---|
| | | 24 hr after infection | 48 hr after infection | 72 hr after infection |
| Antibody 3 (100 ng) | 4.1 | 0 | 0 | 0 |
| Antibody 5 (100 ng) | 5.4 | 0 | 0 | 0 |
| Positive control antibody 4 (100 ng) | 3.9 | 2.1 | 3.1 | 3.2 |
| Negative control antibody (100 ng) | 4.2 | 3.4 | 3.9 | 3.5 |

### Example 12. Evaluation of antibody properties and binding characteristics

Based on the results of measurement of antibody properties such as virus-neutralizing activity, etc., two final antibodies for cell line development were selected, and the properties thereof were evaluated. As evaluation items, aggregate formation evaluation, sequence analysis, disulfide bond maintenance analysis, signal peptide cleavage evaluation, and heavy-chain and light-chain normal binding were measured. As shown in Table 9 below, there were no special issues in the antibody properties.

**[Table 9]**

| Evaluation method | Binding ELISA | DSF evaluation | SEC-HPLC | | | Peptide mapping LC/MS | | | CE-SDS | |
|---|---|---|---|---|---|---|---|---|---|---|
| Evaluation unit | Binding site | Onset (°C) | Main peak (%) | EPAIN (%) | LMW (%) | Cleavage (%) | Bond formation | Cleavage (%) | Intact IgG (%) | Impurity (%) |
| Antibody 3 | MERS-RBD | 57 | 97.5 | 2.5 | 0 | 100 | Normal | H(99.6),L(100) | 80.31 | 19.69 |
| Antibody 5 | MERS-RBD | 57 | 99.61 | 0.39 | 0 | 100 | Normal | H(99.98),L(99.96) | 81.86 | 18.14 |

Also, antigen-binding capacity to MERS-CoV surface protein (RBD) was evaluated (SPR, Surface Plasmon Resonance). As shown in Table 10 below, the neutralizing activity of the antibody and antibody properties were comprehensively judged, and the binding characteristics of the two finally selected antibodies were evaluated in comparison with positive control antibody 4. It was confirmed that antibody 5 had superior binding characteristics compared to positive control antibody 4.

**[Table 10]**

| Sample | ka1 (1/Ms) | kd1 (1/s) | ka2 (1/s) | kd2 (1/s) | Rmax (RU) | Chi2 (RU²) | KD (M) | Average | SD | o/oCV. | %Rel |
|---|---|---|---|---|---|---|---|---|---|---|---|
| Antibody3 | 1.137E+06 | 1.379E-03 | 8.156E-03 | 380E-03 | 77.63 | 1.310 | 1.21E-09 | 1. 03E-09 | 2.55E-10 | 24.7 | 78 |
| | 1.277E+06 | 1.089E-03 | 7.265E-03 | 4.515E-03 | 71.71 | 0.751 | 8.53E-10 | | | | |
| Antibody5 | 2.115E+06 | 2.830E-04 | 2.837E-02 | 2.329E-03 | 87.69 | 0.539 | 1.34E-10 | 1.36E-10 | 2.90E-12 | 2.1 | 589 |
| | 2.060E+06 | 2.841E-04 | 2.420E-02 | 2.142E-03 | 86.82 | 0.463 | 1.38E-10 | | | | |
| Positive control antibody 4 | 1.674E+05 | 1.343E-04 | 9.841E-03 | 8.071E-03 | 75.88 | 0.041 | 8.02E-10 | 8.01E-10 | 1.77E-12 | 0.2 | 100 |
| | 1.723E+05 | 1.378E-04 | 1.241E-02 | 1.082E-02 | 74.87 | 0.031 | 8.00E-10 | | | | |

### Example 13. Evaluation of virus-neutralizing activity (in vivo)

The neutralizing activity of the two antibodies for cell line development against MERS-CoV was evaluated using an animal model. Specifically, the antibody was administered to hDPP4 (human dipeptidyl peptidase 4) receptor-overexpressing mice (TG mice), required for infection of the human body with the virus, and infection with Korean isolate MERS-CoV (MERS-CoV/Korea/KNIH/002_05_2015) was conducted, and thus the preventive and therapeutic efficacies of the two finally selected antibodies were evaluated.

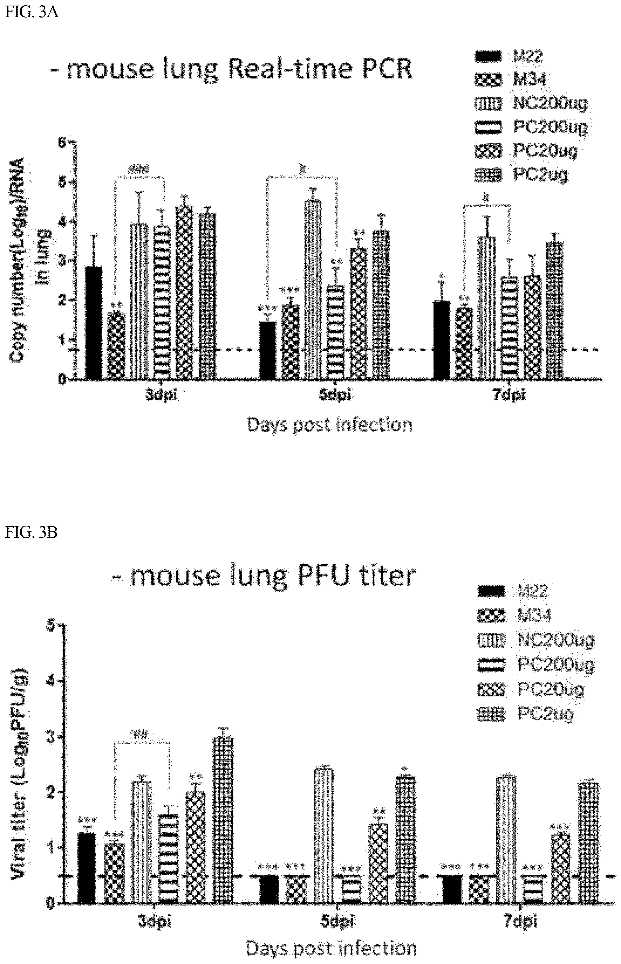

Specifically, the infection conditions of hDPP4 TG mice with MERS-CoV and the effect of positive control antibody 4 were established using antibodies 3 and 5 and the negative control antibody (FIG. 3). For this, animals were infected with the virus, and on the next day, each antibody was intraperitoneally injected thereto in the corresponding dose. A certain number of days after infection, the lung tissue of the mice was extracted, and the virus was quantified through quantitative PCR (FIG. 3A) and a plaque assay (FIG. 3B). It was confirmed in advance that the animal infection experiment was properly progressed and also that antibody 3 and antibody 5 were capable of having superior or similar therapeutic efficacy compared to positive control antibody 4.

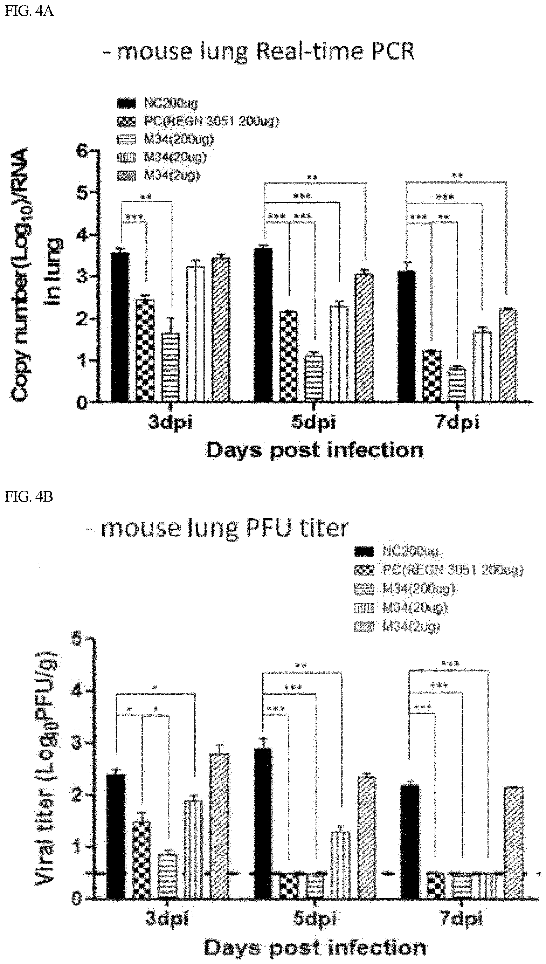

In order to evaluate the preventive efficacy, each of antibody 5, positive control antibody 4 and the negative control antibody was intraperitoneally injected to hDPP4 (human dipeptidyl peptidase 4) receptor-overexpressing mice (hDPP4 TG mouse), required for infection of the human body with MERS-CoV, and on the next day, infection with Korean isolate MERS-CoV (MERS-CoV/Korea/KNIH/002_05_2015) was conducted. A certain number of days after infection, the lung tissue of the mice was extracted, and the virus was quantified (FIG. 4). Based on the MERS-CoV quantification results through quantitative PCR (FIG. 4A) and a plaque assay (FIG. 4B), it was confirmed that antibody 5 had superior preventive efficacy compared to positive control antibody 4.

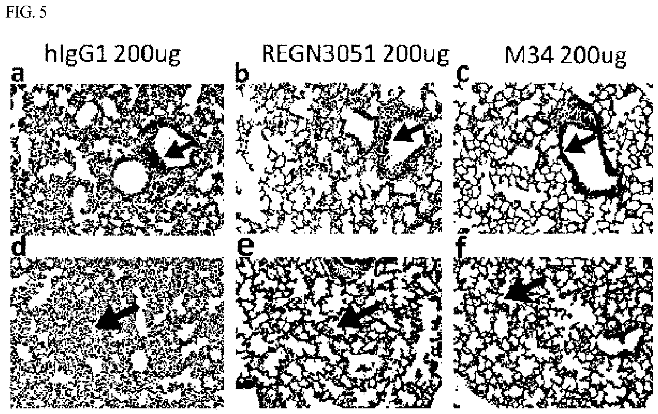

Moreover, the mouse lung was extracted on the 7^{th} day of infection, after which changes in the tissue were directly observed through H&E staining (FIG. 5). Therefore, in the group treated with the negative control antibody, the airway diaphragm was thickened, and many inflammatory cells were introduced, indicating severe histopathological changes ("a" and "d" of FIG. 5). Although the group treated with the positive control antibody was not as severely affected as the group treated with the negative control antibody, the introduction of inflammatory cells and thickened airway diaphragm were confirmed ("b" and "e" of FIG. 5). In contrast, in the group treated with antibody 5, it was confirmed that there were fewer histopathological changes compared to the other treated groups ("c" and "f" of FIG. 5).

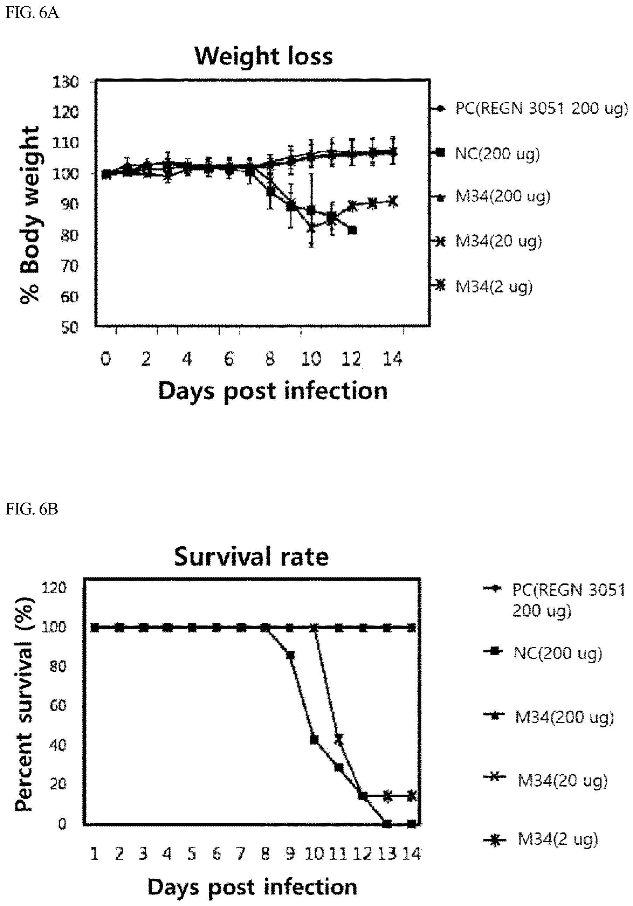

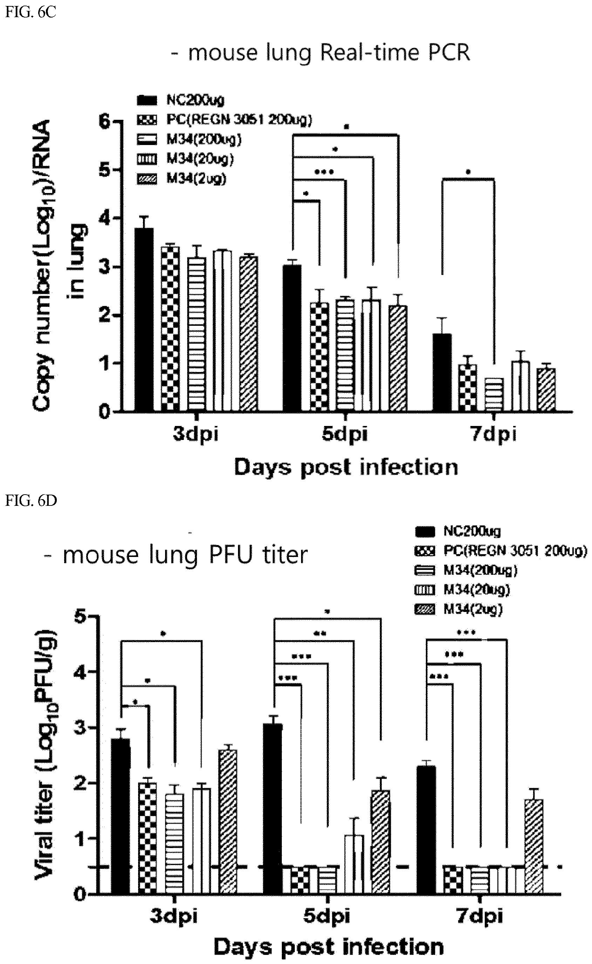

Furthermore, for the evaluation of therapeutic efficacy, animals were infected with the above virus, and on the next day, each of antibody 5, positive control antibody 4 and the negative control antibody was intraperitoneally injected thereto. A certain number of days after infection, the mouse body weight reduction and the mouse survival rate were observed, and also, the mouse lung tissue was extracted and thus the virus was quantified (FIG. 6). Based on the results of evaluation of mouse body weight reduction (FIG. 6A) and mouse survival rate (FIG. 6B), weight loss and mortality were observed only at the lowest dose (2 µg) of the negative control antibody and antibody 5. Also, based on the results of MERS-CoV quantification through quantitative PCR (FIG. 6C) and a plaque assay (FIG. 6D), it was confirmed that antibody 5 had superior or similar therapeutic efficacy compared to positive control antibody 4.

## Claims

1. A neutralizing binding molecule, which binds to a spike protein (S protein) on a surface of a Middle East Respiratory Syndrome - Coronavirus (MERS-CoV).

2. The binding molecule of claim 1, wherein the binding molecule is any one selected from the group consisting of binding molecules i) to vi) below:
i) a binding molecule comprising a) a heavy-chain variable region comprising a CDR1 region of SEQ ID NO: 1, a CDR2 region of SEQ ID NO: 2, and a CDR3 region of SEQ ID NO: 3, and b) a light-chain variable region comprising a CDR1 region of SEQ ID NO: 4, a CDR2 region of SEQ ID NO: 5, and a CDR3 region of SEQ ID NO: 6;
ii) a binding molecule comprising a) a heavy-chain variable region comprising a CDR1 region of SEQ ID NO: 7, a CDR2 region of SEQ ID NO: 8, and a CDR3 region of SEQ ID NO: 9, and b) a light-chain variable region comprising a CDR1 region of SEQ ID NO: 10, a CDR2 region of SEQ ID NO: 11, and a CDR3 region of SEQ ID NO: 12;
iii) a binding molecule comprising a) a heavy-chain variable region comprising a CDR1 region of SEQ ID NO: 13, a CDR2 region of SEQ ID NO: 14, and a CDR3 region of SEQ ID NO: 15, and b) a light-chain variable region comprising a CDR1 region of SEQ ID NO: 16, a CDR2 region of SEQ ID NO: 17, and a CDR3 region of SEQ ID NO: 18;
iv) a binding molecule comprising a) a heavy-chain variable region comprising a CDR1 region of SEQ ID NO: 19, a CDR2 region of SEQ ID NO: 20, and a CDR3 region of SEQ ID NO: 21, and b) a light-chain variable region comprising a CDR1 region of SEQ ID NO: 22, a CDR2 region of SEQ ID NO: 23, and a CDR3 region of SEQ ID NO: 24;
v) a binding molecule comprising a) a heavy-chain variable region comprising a CDR1 region of SEQ ID NO: 25, a CDR2 region of SEQ ID NO: 26, and a CDR3 region of SEQ ID NO: 27, and b) a light-chain variable region comprising a CDR1 region of SEQ ID NO: 28, a CDR2 region of SEQ ID NO: 29, and a CDR3 region of SEQ ID NO: 30; and
vi) a binding molecule comprising a) a heavy-chain variable region comprising a CDR1 region of SEQ ID NO: 31, a CDR2 region of SEQ ID NO: 32, and a CDR3 region of SEQ ID NO: 33, and b) a light-chain variable region comprising a CDR1 region of SEQ ID NO: 34, a CDR2 region of SEQ ID NO: 35, and a CDR3 region of SEQ ID NO: 36.

3. The binding molecule of claim 1, wherein the binding molecule is any one selected from the group consisting of binding molecules i) to vi) below:
i) a binding molecule comprising a) a heavy-chain variable region having a sequence identity of 95% or more to a heavy-chain variable region of a polypeptide sequence of SEQ ID NO: 37, and b) a light-chain variable region having a sequence identity of 95% or more to a light-chain variable region of a polypeptide sequence of SEQ ID NO: 38;
ii) a binding molecule comprising a) a heavy-chain variable region having a sequence identity of 95% or more to a heavy-chain variable region of a polypeptide sequence of SEQ ID NO: 39, and b) a light-chain variable region having a sequence identity of 95% or more to a light-chain variable region of a polypeptide sequence of SEQ ID NO: 40;
iii) a binding molecule comprising a) a heavy-chain variable region having a sequence identity of 95% or more to a heavy-chain variable region of a polypeptide sequence of SEQ ID NO: 41, and b) a light-chain variable region having a sequence identity of 95% or more to a light-chain variable region of a polypeptide sequence of SEQ ID NO: 42;
iv) a binding molecule comprising a) a heavy-chain variable region having a sequence identity of 95% or more to a heavy-chain variable region of a polypeptide sequence of SEQ ID NO: 43, and b) a light-chain variable region having a sequence identity of 95% or more to a light-chain variable region of a polypeptide sequence of SEQ ID NO: 44;
v) a binding molecule comprising a) a heavy-chain variable region having a sequence identity of 95% or more to a heavy-chain variable region of a polypeptide sequence of SEQ ID NO: 45, and b) a light-chain variable region having a sequence identity of 95% or more to a light-chain variable region of a polypeptide sequence of SEQ ID NO: 46; and
vi) a binding molecule comprising a) a heavy-chain variable region having a sequence identity of 95% or more to a heavy-chain variable region of a polypeptide sequence of SEQ ID NO: 47, and b) a light-chain variable region having a sequence identity of 95% or more to a light-chain variable region of a polypeptide sequence of SEQ ID NO: 48.

4. The binding molecule of any one of claims 1 to 3, wherein the binding molecule is a Fab fragment, a Fv fragment, a diabody, a chimeric antibody, a humanized antibody, or a human antibody.

5. An immunoconjugate in which at least one tag is additionally bound to the binding molecule of any one of claims 1 to 3.

6. A nucleic acid molecule encoding the binding molecule of any one of claims 1 to 3.

7. An expression vector into which the nucleic acid molecule of claim 6 is inserted.

8. A cell line in which the expression vector of claim 7 is transformed into a host cell to produce a binding molecule that binds to MERS-CoV and thus has neutralizing activity.

9. The cell line of claim 8, wherein the host cell is any one selected from the group consisting of a CHO cell, a F2N cell, a COS cell, a BHK cell, a Bowes melanoma cell, a HeLa cell, a 911 cell, a HT1080 cell, an A549 cell, a HEK 293 cell and a HEK293T cell.

10. A composition for preventing or treating MERS-CoV infection comprising the binding molecule of any one of claims 1 to 3.

11. The composition of claim 10, wherein the composition is a sterile injectable solution, a lyophilized formulation, a pre-filled syringe solution, an oral formulation, a formulation for external use, or a suppository.

12. A method of diagnosing, preventing or treating a disease caused by MERS-CoV infection comprising administering the composition of claim 10 or 11 in a therapeutically effective amount to a subject having a disease caused by MERS-CoV infection.

13. A kit for diagnosing MERS-CoV comprising the binding molecule of any one of claims 1 to 3.


<110>    CELLTRION INC.

<120>    A binding molecules able to neutralize middle east
         respiratory-syndrome coronavirus

<130>    CPP2018069PCT

<150>    KR 10/20180011776
<151>    2018-01-31

<150>    KR 10/20180108125
<151>    2018-09-11

<150>    KR 10-2018-0138228
<151>    2018-11-12

<160>    48

<170>    KoPatentIn 3.0

<210>    1
<211>    10
<212>    PRT
<213>    Artificial Sequence

<220>
<223>    Antibody 1 heavy chain CDR1


<400>    1
Gly Gly Thr Phe Ser Ser Tyr Val Ile Ser
  1               5                   10


<210>    2
<211>    17
<212>    PRT
<213>    Artificial Sequence

<220>
<223>    Antibody 1 heavy chain CDR2


<400>    2
Arg Ile Ile Pro Ile Phe Gly Thr Val Lys Tyr Ala Gln Lys Phe Gln
  1               5                   10                  15

Gly



<210>    3
<211>    17
<212>    PRT
<213>    Artificial Sequence

<220>
<223>    Antibody 1 heavy chain CDR3


<400>    3
Asp Leu Pro Gly Asp Ser Arg Asp Gly Tyr Asn Tyr Asp Ala Phe Asp
  1               5                   10                  15

Ile

<210> 4
<211> 13
<212> PRT
<213> Artificial Sequence

<220>
<223> Antibody 1 light chain CDR1

<400> 4
Ser Gly Ser Ser Ser Asn Ile Gly Asn Asn Tyr Val Ser
1               5                   10

<210> 5
<211> 7
<212> PRT
<213> Artificial Sequence

<220>
<223> Antibody 1 light chain CDR2

<400> 5
Asp Asn Asn Lys Arg Pro Ser
1               5

<210> 6
<211> 11
<212> PRT
<213> Artificial Sequence

<220>
<223> Antibody 1 light chain CDR3

<400> 6
Gly Thr Trp Asp Ser Ser Leu Asn Ala Gly Val
1               5                   10

<210> 7
<211> 10
<212> PRT
<213> Artificial Sequence

<220>
<223> Antibody 2 heavy chain CDR1

<400> 7
Gly Gly Thr Phe Ser Ser Tyr Ala Ile Ser
1               5                   10

<210> 8
<211> 17

<212>     PRT
<213>     Artificial Sequence

<220>
<223>     Antibody 2 heavy chain CDR2


<400>     8
Arg Ile Ile Pro Ile Phe Gly Ile Ala Lys Tyr Ala Gln Lys Phe Gln
1               5                   10                  15

Gly




<210>     9
<211>     17
<212>     PRT
<213>     Artificial Sequence

<220>
<223>     Antibody 2 heavy chain CDR3


<400>     9
Asp Leu Pro Gly Asp Ser Arg Asp Gly Tyr Asn Tyr Asp Ala Phe Asp
1               5                   10                  15

Ile




<210>     10
<211>     13
<212>     PRT
<213>     Artificial Sequence

<220>
<223>     Antibody 2 light chain CDR1


<400>     10
Ser Gly Ser Ser Ser Asn Ile Gly Asn Asn Phe Val Ser
1               5                   10


<210>     11
<211>     7
<212>     PRT
<213>     Artificial Sequence

<220>
<223>     Antibody 2 light chain CDR2


<400>     11
Asp Asn Asp Lys Arg Pro Ser
1               5


<210>     12
<211>     11
<212>     PRT

<213>    Artificial Sequence

<220>
<223>    Antibody 2 light chain CDR3


<400>    12
Ala Thr Trp Asp Arg Ser Leu Ser Val Glu Leu
1               5                   10


<210>    13
<211>    12
<212>    PRT
<213>    Artificial Sequence

<220>
<223>    Antibody 3 heavy chain CDR1


<400>    13
Gly Gly Ser Ile Asn Ser Arg Ser Tyr Tyr Trp Gly
1               5                   10


<210>    14
<211>    16
<212>    PRT
<213>    Artificial Sequence

<220>
<223>    Antibody 3 heavy chain CDR2


<400>    14
Ser Ile Tyr Tyr Ser Gly Ser Thr Tyr Tyr Asn Pro Ser Leu Lys Ser
1               5                   10                  15


<210>    15
<211>    19
<212>    PRT
<213>    Artificial Sequence

<220>
<223>    Antibody 3 heavy chain CDR3


<400>    15
Ala Asp Arg Glu His Val Trp Gly Ser Tyr Asp Gly Pro Gly Ser Pro
1               5                   10                  15

Phe Asp Tyr


<210>    16
<211>    13
<212>    PRT
<213>    Artificial Sequence

<220>

<223>    Antibody 3 light chain CDR1

<400>    16
Ser Gly Ser Ser Ser Asn Ile Gly Ser Asn Thr Val Asn
1               5                   10

<210>    17
<211>    7
<212>    PRT
<213>    Artificial Sequence

<220>
<223>    Antibody 3 light chain CDR2

<400>    17
Ser Ser Asn Gln Arg Pro Ser
1               5

<210>    18
<211>    11
<212>    PRT
<213>    Artificial Sequence

<220>
<223>    Antibody 3 light chain CDR3

<400>    18
Ala Ala Trp Asp Asp Ser Leu Asn Gly Leu Val
1               5                   10

<210>    19
<211>    12
<212>    PRT
<213>    Artificial Sequence

<220>
<223>    Antibody 4 heavy chain CDR1

<400>    19
Gly Gly Ser Ile Asn Ser Arg Ser Tyr Tyr Trp Gly
1               5                   10

<210>    20
<211>    16
<212>    PRT
<213>    Artificial Sequence

<220>
<223>    Antibody 4 heavy chain CDR2

<400>    20
Ser Ile Tyr Tyr Ser Gly Ser Thr Tyr Tyr Asn Pro Ser Leu Lys Ser
1               5                   10                  15

<210>    21
<211>    19
<212>    PRT
<213>    Artificial Sequence

<220>
<223>    Antibody 4 heavy chain CDR3


<400>    21
Ala Asp Arg Glu Tyr Val Trp Gly Ser Tyr Asp Gly Pro Gly Ser Pro
1               5                   10                  15

Phe Asp Tyr




<210>    22
<211>    13
<212>    PRT
<213>    Artificial Sequence

<220>
<223>    Antibody 4 light chain CDR1


<400>    22
Ser Gly Ser Ser Ser Asn Ile Gly Asn Asn Ala Val Asn
1               5                   10


<210>    23
<211>    7
<212>    PRT
<213>    Artificial Sequence

<220>
<223>    Antibody 4 light chain CDR2


<400>    23
Gly Asp His Gln Arg Pro Ser
1               5


<210>    24
<211>    11
<212>    PRT
<213>    Artificial Sequence

<220>
<223>    Antibody 4 light chain CDR3


<400>    24
Ala Ala Trp Asp Asp Ser Leu Asn Gly Val Val
1               5                   10


<210>    25
<211>    10

<212>     PRT
<213>     Artificial Sequence

<220>
<223>     Antibody 5 heavy chain CDR1


<400>     25
Gly Gly Thr Phe Ser Ser Tyr Ala Ile Ser
1               5                   10


<210>     26
<211>     17
<212>     PRT
<213>     Artificial Sequence

<220>
<223>     Antibody 5 heavy chain CDR2


<400>     26
Arg Ile Ile Pro Ile Phe Gly Ile Ala Lys Tyr Ala Gln Lys Phe Gln
1               5                   10                  15

Gly



<210>     27
<211>     15
<212>     PRT
<213>     Artificial Sequence

<220>
<223>     Antibody 5 heavy chain CDR3


<400>     27
Arg Ser Gly Asp Tyr Ser Gly Ser Ser Gly Leu Ala Phe Asp Ile
1               5                   10                  15


<210>     28
<211>     12
<212>     PRT
<213>     Artificial Sequence

<220>
<223>     Antibody 5 light chain CDR1


<400>     28
Arg Ala Ser Gln Ser Val Ser Ser Ser Tyr Leu Ala
1               5                   10


<210>     29
<211>     7
<212>     PRT
<213>     Artificial Sequence

<220>

<223>    Antibody 5 light chain CDR2

<400>    29
Gly Ala Ser Ser Arg Ala Thr
1               5

<210>    30
<211>    9
<212>    PRT
<213>    Artificial Sequence

<220>
<223>    Antibody 5 light chain CDR3

<400>    30
Gln Gln Tyr Gly Ser Ser Pro Tyr Thr
1               5

<210>    31
<211>    10
<212>    PRT
<213>    Artificial Sequence

<220>
<223>    Antibody 6 heavy chain CDR1

<400>    31
Gly Phe Thr Phe Asp Asp Tyr Ala Met His
1               5                   10

<210>    32
<211>    17
<212>    PRT
<213>    Artificial Sequence

<220>
<223>    Antibody 6 heavy chain CDR2

<400>    32
Gly Ile Ser Trp Asn Ser Gly Thr Ile Gly Tyr Ala Asp Ser Val Lys
1               5                   10                  15

Gly

<210>    33
<211>    12
<212>    PRT
<213>    Artificial Sequence

<220>
<223>    Antibody 6 heavy chain CDR3

<400>    33

Asp Trp Ser Ser Gly Trp Ser Ser Pro Leu Asp Tyr
1               5                   10

<210>    34
<211>    14
<212>    PRT
<213>    Artificial Sequence

<220>
<223>    Antibody 6 light chain CDR1

<400>    34
Thr Gly Ser Ser Ser Asn Ile Gly Ala Gly Tyr Asp Val His
1               5                   10

<210>    35
<211>    7
<212>    PRT
<213>    Artificial Sequence

<220>
<223>    Antibody 6 light chain CDR2

<400>    35
Gly Asn Ser Asn Arg Pro Ser
1               5

<210>    36
<211>    11
<212>    PRT
<213>    Artificial Sequence

<220>
<223>    Antibody 6 light chain CDR3

<400>    36
Gln Ser Tyr Asp Ser Ser Leu Ser Gly Val Val
1               5                   10

<210>    37
<211>    466
<212>    PRT
<213>    Artificial Sequence

<220>
<223>    Antibody 1 heavy chain variable region

<400>    37
Gln Val Gln Leu Val Gln Ser Gly Ala Glu Val Lys Lys Pro Gly Ser
1               5                   10                  15

Ser Val Lys Val Ser Cys Lys Ala Ser Gly Gly Thr Phe Ser Ser Tyr
            20                  25                  30

Val Ile Ser Trp Val Arg Gln Ala Pro Gly Gln Gly Leu Glu Trp Val

35                  40                  45

Gly Arg Ile Ile Pro Ile Phe Gly Thr Val Lys Tyr Ala Gln Lys Phe
    50                  55                  60

Gln Gly Arg Val Thr Ile Thr Ala Asp Lys Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Arg Asp Leu Pro Gly Asp Ser Arg Asp Gly Tyr Asn Tyr Asp Ala
            100                 105                 110

Phe Asp Ile Trp Gly Gln Gly Thr Leu Val Thr Val Ser Leu Ala Ser
        115                 120                 125

Thr Lys Gly Pro Ser Val Thr Ser Ala Ser Thr Lys Gly Pro Ser Val
    130                 135                 140

Phe Pro Leu Ala Pro Ser Ser Lys Ser Thr Ser Gly Gly Thr Ala Ala
145                 150                 155                 160

Leu Gly Cys Leu Val Lys Asp Tyr Phe Pro Glu Pro Val Thr Val Ser
                165                 170                 175

Trp Asn Ser Gly Ala Leu Thr Ser Gly Val His Thr Phe Pro Ala Val
            180                 185                 190

Leu Gln Ser Ser Gly Leu Tyr Ser Leu Ser Ser Val Val Thr Val Pro
        195                 200                 205

Ser Ser Ser Leu Gly Thr Gln Thr Tyr Ile Cys Asn Val Asn His Lys
    210                 215                 220

Pro Ser Asn Thr Lys Val Asp Lys Lys Val Glu Pro Lys Ser Cys Asp
225                 230                 235                 240

Lys Thr His Thr Cys Pro Pro Cys Pro Ala Pro Glu Leu Leu Gly Gly
                245                 250                 255

Pro Ser Val Phe Leu Phe Pro Pro Lys Pro Lys Asp Thr Leu Met Ile
            260                 265                 270

Ser Arg Thr Pro Glu Val Thr Cys Val Val Val Asp Val Ser His Glu
        275                 280                 285

Asp Pro Glu Val Lys Phe Asn Trp Tyr Val Asp Gly Val Glu Val His
    290                 295                 300

Asn Ala Lys Thr Lys Pro Arg Glu Glu Gln Tyr Asn Ser Thr Tyr Arg
305                 310                 315                 320

Val Val Ser Val Leu Thr Val Leu His Gln Asp Trp Leu Asn Gly Lys
                325                 330                 335

Glu Tyr Lys Cys Lys Val Ser Asn Lys Ala Leu Pro Ala Pro Ile Glu
            340                 345                 350

Lys Thr Ile Ser Lys Ala Lys Gly Gln Pro Arg Glu Pro Gln Val Tyr
        355                 360                 365

Thr Leu Pro Pro Ser Arg Glu Glu Met Thr Lys Asn Gln Val Ser Leu

370                 375                 380

Thr Cys Leu Val Lys Gly Phe Tyr Pro Ser Asp Ile Ala Val Glu Trp
385                 390                 395                 400

Glu Ser Asn Gly Gln Pro Glu Asn Asn Tyr Lys Thr Thr Pro Pro Val
                405                 410                 415

Leu Asp Ser Asp Gly Ser Phe Phe Leu Tyr Ser Lys Leu Thr Val Asp
            420                 425                 430

Lys Ser Arg Trp Gln Gln Gly Asn Val Phe Ser Cys Ser Val Met His
        435                 440                 445

Glu Ala Leu His Asn His Tyr Thr Gln Lys Ser Leu Ser Leu Ser Pro
    450                 455                 460

Gly Lys
465

<210>    38
<211>    219
<212>    PRT
<213>    Artificial Sequence

<220>
<223>    Antibody 1 light chain variable region

<400>    38
Gly Glu Leu Glu Leu Thr Gln Pro Pro Ser Val Ser Ala Ala Pro Gly
1               5                   10                  15

Gln Lys Val Thr Ile Ser Cys Ser Gly Ser Ser Ser Asn Ile Gly Asn
            20                  25                  30

Asn Tyr Val Ser Trp Tyr Gln Gln Leu Pro Gly Thr Ala Pro Lys Leu
        35                  40                  45

Leu Ile Tyr Asp Asn Asn Lys Arg Pro Ser Gly Ile Pro Asp Arg Phe
    50                  55                  60

Ser Gly Ser Lys Ser Gly Thr Ser Ala Thr Leu Gly Ile Thr Gly Leu
65                  70                  75                  80

Gln Thr Gly Asp Glu Ala Asp Tyr Tyr Cys Gly Thr Trp Asp Ser Ser
                85                  90                  95

Leu Asn Ala Gly Val Phe Gly Gly Gly Thr Glu Leu Thr Val Leu Gly
            100                 105                 110

Arg Thr Val Ala Ala Pro Ser Val Phe Ile Phe Pro Pro Ser Asp Glu
        115                 120                 125

Gln Leu Lys Ser Gly Thr Ala Ser Val Val Cys Leu Leu Asn Asn Phe
    130                 135                 140

Tyr Pro Arg Glu Ala Lys Val Gln Trp Lys Val Asp Asn Ala Leu Gln
145                 150                 155                 160

Ser Gly Asn Ser Gln Glu Ser Val Thr Glu Gln Asp Ser Lys Asp Ser
                165                 170                 175

Thr Tyr Ser Leu Ser Ser Thr Leu Thr Leu Ser Lys Ala Asp Tyr Glu
            180                 185                 190

Lys His Lys Val Tyr Ala Cys Glu Val Thr His Gln Gly Leu Ser Ser
        195                 200                 205

Pro Val Thr Lys Ser Phe Asn Arg Gly Glu Cys
    210                 215

<210> 39
<211> 466
<212> PRT
<213> Artificial Sequence

<220>
<223> Antibody 2 heavy chain variable region

<400> 39
Glu Val Gln Leu Val Gln Ser Gly Ala Glu Val Lys Lys Pro Gly Ser
1               5                   10                  15

Ser Val Lys Val Ser Cys Lys Ala Ser Gly Gly Thr Phe Ser Ser Tyr
            20                  25                  30

Ala Ile Ser Trp Val Arg Gln Ala Pro Gly Gln Gly Leu Glu Trp Met
        35                  40                  45

Gly Arg Ile Ile Pro Ile Phe Gly Ile Ala Lys Tyr Ala Gln Lys Phe
    50                  55                  60

Gln Gly Arg Val Thr Ile Thr Ala Asp Lys Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Arg Asp Leu Pro Gly Asp Ser Arg Asp Gly Tyr Asn Tyr Asp Ala
            100                 105                 110

Phe Asp Ile Trp Gly Gln Gly Thr Met Val Thr Val Ser Ser Ala Ser
        115                 120                 125

Thr Lys Gly Pro Ser Val Thr Ser Ala Ser Thr Lys Gly Pro Ser Val
    130                 135                 140

Phe Pro Leu Ala Pro Ser Ser Lys Ser Thr Ser Gly Gly Thr Ala Ala
145                 150                 155                 160

Leu Gly Cys Leu Val Lys Asp Tyr Phe Pro Glu Pro Val Thr Val Ser
                165                 170                 175

Trp Asn Ser Gly Ala Leu Thr Ser Gly Val His Thr Phe Pro Ala Val
            180                 185                 190

Leu Gln Ser Ser Gly Leu Tyr Ser Leu Ser Ser Val Val Thr Val Pro
        195                 200                 205

Ser Ser Ser Leu Gly Thr Gln Thr Tyr Ile Cys Asn Val Asn His Lys
    210                 215                 220

Pro Ser Asn Thr Lys Val Asp Lys Lys Val Glu Pro Lys Ser Cys Asp
225                 230                 235                 240

Lys Thr His Thr Cys Pro Pro Cys Pro Ala Pro Glu Leu Leu Gly Gly
                245                 250                 255

Pro Ser Val Phe Leu Phe Pro Pro Lys Pro Lys Asp Thr Leu Met Ile
            260                 265                 270

Ser Arg Thr Pro Glu Val Thr Cys Val Val Val Asp Val Ser His Glu
        275                 280                 285

Asp Pro Glu Val Lys Phe Asn Trp Tyr Val Asp Gly Val Glu Val His
    290                 295                 300

Asn Ala Lys Thr Lys Pro Arg Glu Glu Gln Tyr Asn Ser Thr Tyr Arg
305                 310                 315                 320

Val Val Ser Val Leu Thr Val Leu His Gln Asp Trp Leu Asn Gly Lys
                325                 330                 335

Glu Tyr Lys Cys Lys Val Ser Asn Lys Ala Leu Pro Ala Pro Ile Glu
            340                 345                 350

Lys Thr Ile Ser Lys Ala Lys Gly Gln Pro Arg Glu Pro Gln Val Tyr
        355                 360                 365

Thr Leu Pro Pro Ser Arg Glu Glu Met Thr Lys Asn Gln Val Ser Leu
    370                 375                 380

Thr Cys Leu Val Lys Gly Phe Tyr Pro Ser Asp Ile Ala Val Glu Trp
385                 390                 395                 400

Glu Ser Asn Gly Gln Pro Glu Asn Asn Tyr Lys Thr Thr Pro Pro Val
                405                 410                 415

Leu Asp Ser Asp Gly Ser Phe Phe Leu Tyr Ser Lys Leu Thr Val Asp
            420                 425                 430

Lys Ser Arg Trp Gln Gln Gly Asn Val Phe Ser Cys Ser Val Met His
        435                 440                 445

Glu Ala Leu His Asn His Tyr Thr Gln Lys Ser Leu Ser Leu Ser Pro
    450                 455                 460

Gly Lys
465

<210>  40
<211>  219
<212>  PRT
<213>  Artificial Sequence

<220>
<223>  Antibody 2 light chain variable region

<400>  40
Gly Glu Leu Val Leu Thr Gln Pro Pro Ser Val Ser Ala Ala Pro Gly
1               5                   10                  15

Gln Lys Val Thr Ile Ser Cys Ser Gly Ser Ser Ser Asn Ile Gly Asn

                20                  25                  30

Asn Phe Val Ser Trp Tyr Gln Gln Leu Pro Gly Thr Ala Pro Lys Leu
        35                  40                  45

Leu Ile Tyr Asp Asn Asp Lys Arg Pro Ser Gly Ile Pro Asp Arg Phe
    50                  55                  60

Ser Gly Ser Lys Ser Gly Thr Ser Ala Thr Leu Gly Ile Thr Gly Leu
65                  70                  75                  80

Gln Thr Gly Asp Glu Ala Asp Tyr Tyr Cys Ala Thr Trp Asp Arg Ser
                85                  90                  95

Leu Ser Val Glu Leu Phe Gly Gly Gly Thr Lys Val Thr Val Leu Gly
            100                 105                 110

Arg Thr Val Ala Ala Pro Ser Val Phe Ile Phe Pro Pro Ser Asp Glu
        115                 120                 125

Gln Leu Lys Ser Gly Thr Ala Ser Val Val Cys Leu Leu Asn Asn Phe
    130                 135                 140

Tyr Pro Arg Glu Ala Lys Val Gln Trp Lys Val Asp Asn Ala Leu Gln
145                 150                 155                 160

Ser Gly Asn Ser Gln Glu Ser Val Thr Glu Gln Asp Ser Lys Asp Ser
                165                 170                 175

Thr Tyr Ser Leu Ser Ser Thr Leu Thr Leu Ser Lys Ala Asp Tyr Glu
            180                 185                 190

Lys His Lys Val Tyr Ala Cys Glu Val Thr His Gln Gly Leu Ser Ser
        195                 200                 205

Pro Val Thr Lys Ser Phe Asn Arg Gly Glu Cys
    210                 215


<210>   41
<211>   469
<212>   PRT
<213>   Artificial Sequence

<220>
<223>   Antibody 3 heavy chain variable region


<400>   41
Gln Val Gln Leu Gln Glu Ser Gly Pro Gly Leu Val Lys Pro Ser Glu
1               5                   10                  15

Thr Leu Ser Leu Thr Cys Thr Val Ser Gly Gly Ser Ile Asn Ser Arg
            20                  25                  30

Ser Tyr Tyr Trp Gly Trp Ile Arg Gln Pro Pro Gly Lys Gly Leu Glu
        35                  40                  45

Trp Ile Gly Ser Ile Tyr Tyr Ser Gly Ser Thr Tyr Tyr Asn Pro Ser
    50                  55                  60

Leu Lys Ser Arg Val Thr Ile Ser Val Asp Thr Ser Lys Asn Gln Phe
65                  70                  75                  80

Ser Leu Lys Leu Arg Ser Val Thr Ala Ala Asp Thr Ala Val Tyr Tyr
                85                  90                  95

Cys Ala Arg Ala Asp Arg Glu His Val Trp Gly Ser Tyr Asp Gly Pro
            100                 105                 110

Gly Ser Pro Phe Asp Tyr Trp Gly Gln Gly Thr Leu Val Thr Val Ser
        115                 120                 125

Ser Ala Ser Thr Lys Gly Pro Ser Val Thr Ser Ala Ser Thr Lys Gly
    130                 135                 140

Pro Ser Val Phe Pro Leu Ala Pro Ser Ser Lys Ser Thr Ser Gly Gly
145                 150                 155                 160

Thr Ala Ala Leu Gly Cys Leu Val Lys Asp Tyr Phe Pro Glu Pro Val
                165                 170                 175

Thr Val Ser Trp Asn Ser Gly Ala Leu Thr Ser Gly Val His Thr Phe
            180                 185                 190

Pro Ala Val Leu Gln Ser Ser Gly Leu Tyr Ser Leu Ser Ser Val Val
        195                 200                 205

Thr Val Pro Ser Ser Ser Leu Gly Thr Gln Thr Tyr Ile Cys Asn Val
    210                 215                 220

Asn His Lys Pro Ser Asn Thr Lys Val Asp Lys Lys Val Glu Pro Lys
225                 230                 235                 240

Ser Cys Asp Lys Thr His Thr Cys Pro Pro Cys Pro Ala Pro Glu Leu
                245                 250                 255

Leu Gly Gly Pro Ser Val Phe Leu Phe Pro Pro Lys Pro Lys Asp Thr
            260                 265                 270

Leu Met Ile Ser Arg Thr Pro Glu Val Thr Cys Val Val Val Asp Val
        275                 280                 285

Ser His Glu Asp Pro Glu Val Lys Phe Asn Trp Tyr Val Asp Gly Val
    290                 295                 300

Glu Val His Asn Ala Lys Thr Lys Pro Arg Glu Glu Gln Tyr Asn Ser
305                 310                 315                 320

Thr Tyr Arg Val Val Ser Val Leu Thr Val Leu His Gln Asp Trp Leu
                325                 330                 335

Asn Gly Lys Glu Tyr Lys Cys Lys Val Ser Asn Lys Ala Leu Pro Ala
            340                 345                 350

Pro Ile Glu Lys Thr Ile Ser Lys Ala Lys Gly Gln Pro Arg Glu Pro
        355                 360                 365

Gln Val Tyr Thr Leu Pro Pro Ser Arg Glu Glu Met Thr Lys Asn Gln
    370                 375                 380

Val Ser Leu Thr Cys Leu Val Lys Gly Phe Tyr Pro Ser Asp Ile Ala
385                 390                 395                 400

Val Glu Trp Glu Ser Asn Gly Gln Pro Glu Asn Asn Tyr Lys Thr Thr
                405                 410                 415

Pro Pro Val Leu Asp Ser Asp Gly Ser Phe Phe Leu Tyr Ser Lys Leu
            420                 425                 430

Thr Val Asp Lys Ser Arg Trp Gln Gln Gly Asn Val Phe Ser Cys Ser
        435                 440                 445

Val Met His Glu Ala Leu His Asn His Tyr Thr Gln Lys Ser Leu Ser
    450                 455                 460

Leu Ser Pro Gly Lys
465

<210> 42
<211> 219
<212> PRT
<213> Artificial Sequence

<220>
<223> Antibody 3 light chain variable region

<400> 42
Gly Glu Leu Glu Leu Thr Gln Pro Pro Ser Val Ser Gly Thr Pro Gly
1               5                   10                  15

Gln Arg Val Thr Ile Ser Cys Ser Gly Ser Ser Ser Asn Ile Gly Ser
            20                  25                  30

Asn Thr Val Asn Trp Tyr Gln Gln Phe Pro Gly Thr Ala Pro Lys Leu
        35                  40                  45

Leu Ile Tyr Ser Ser Asn Gln Arg Pro Ser Gly Val Pro Asp Arg Phe
    50                  55                  60

Ser Gly Ser Lys Ser Gly Thr Ser Ala Ser Leu Ala Ile Ser Gly Leu
65                  70                  75                  80

Gln Ser Gln Asp Glu Ala Asp Tyr His Cys Ala Ala Trp Asp Asp Ser
                85                  90                  95

Leu Asn Gly Leu Val Phe Gly Gly Gly Thr Gln Leu Thr Val Leu Gly
            100                 105                 110

Arg Thr Val Ala Ala Pro Ser Val Phe Ile Phe Pro Pro Ser Asp Glu
        115                 120                 125

Gln Leu Lys Ser Gly Thr Ala Ser Val Val Cys Leu Leu Asn Asn Phe
    130                 135                 140

Tyr Pro Arg Glu Ala Lys Val Gln Trp Lys Val Asp Asn Ala Leu Gln
145                 150                 155                 160

Ser Gly Asn Ser Gln Glu Ser Val Thr Glu Gln Asp Ser Lys Asp Ser
                165                 170                 175

Thr Tyr Ser Leu Ser Ser Thr Leu Thr Leu Ser Lys Ala Asp Tyr Glu
            180                 185                 190

Lys His Lys Val Tyr Ala Cys Glu Val Thr His Gln Gly Leu Ser Ser
        195                 200                 205

Pro Val Thr Lys Ser Phe Asn Arg Gly Glu Cys
    210                 215

<210>   43
<211>   469
<212>   PRT
<213>   Artificial Sequence

<220>
<223>   Antibody 4 heavy chain variable region

<400>   43
Gln Val Gln Leu Gln Glu Ser Gly Pro Gly Leu Val Lys Pro Ser Glu
1               5                   10                  15

Thr Leu Ser Leu Thr Cys Thr Val Ser Gly Gly Ser Ile Asn Ser Arg
            20                  25                  30

Ser Tyr Tyr Trp Gly Trp Ile Arg Gln Pro Pro Gly Lys Gly Leu Glu
        35                  40                  45

Trp Ile Gly Ser Ile Tyr Tyr Ser Gly Ser Thr Tyr Tyr Asn Pro Ser
    50                  55                  60

Leu Lys Ser Arg Val Thr Ile Ser Val Asp Thr Ser Lys Asn Gln Phe
65                  70                  75                  80

Ser Leu Lys Leu Arg Ser Val Thr Ala Ala Asp Thr Ala Val Tyr Tyr
                85                  90                  95

Cys Ala Arg Ala Asp Arg Glu Tyr Val Trp Gly Ser Tyr Asp Gly Pro
            100                 105                 110

Gly Ser Pro Phe Asp Tyr Trp Gly Gln Gly Thr Leu Val Thr Val Ser
        115                 120                 125

Ser Ala Ser Thr Lys Gly Pro Ser Val Thr Ser Ala Ser Thr Lys Gly
    130                 135                 140

Pro Ser Val Phe Pro Leu Ala Pro Ser Ser Lys Ser Thr Ser Gly Gly
145                 150                 155                 160

Thr Ala Ala Leu Gly Cys Leu Val Lys Asp Tyr Phe Pro Glu Pro Val
                165                 170                 175

Thr Val Ser Trp Asn Ser Gly Ala Leu Thr Ser Gly Val His Thr Phe
            180                 185                 190

Pro Ala Val Leu Gln Ser Ser Gly Leu Tyr Ser Leu Ser Ser Val Val
        195                 200                 205

Thr Val Pro Ser Ser Ser Leu Gly Thr Gln Thr Tyr Ile Cys Asn Val
    210                 215                 220

Asn His Lys Pro Ser Asn Thr Lys Val Asp Lys Lys Val Glu Pro Lys
225                 230                 235                 240

Ser Cys Asp Lys Thr His Thr Cys Pro Pro Cys Pro Ala Pro Glu Leu
                245                 250                 255

Leu Gly Gly Pro Ser Val Phe Leu Phe Pro Pro Lys Pro Lys Asp Thr

260                 265                 270

Leu Met Ile Ser Arg Thr Pro Glu Val Thr Cys Val Val Val Asp Val
        275                 280                 285

Ser His Glu Asp Pro Glu Val Lys Phe Asn Trp Tyr Val Asp Gly Val
    290                 295                 300

Glu Val His Asn Ala Lys Thr Lys Pro Arg Glu Glu Gln Tyr Asn Ser
305                 310                 315                 320

Thr Tyr Arg Val Val Ser Val Leu Thr Val Leu His Gln Asp Trp Leu
                325                 330                 335

Asn Gly Lys Glu Tyr Lys Cys Lys Val Ser Asn Lys Ala Leu Pro Ala
            340                 345                 350

Pro Ile Glu Lys Thr Ile Ser Lys Ala Lys Gly Gln Pro Arg Glu Pro
        355                 360                 365

Gln Val Tyr Thr Leu Pro Pro Ser Arg Glu Glu Met Thr Lys Asn Gln
    370                 375                 380

Val Ser Leu Thr Cys Leu Val Lys Gly Phe Tyr Pro Ser Asp Ile Ala
385                 390                 395                 400

Val Glu Trp Glu Ser Asn Gly Gln Pro Glu Asn Asn Tyr Lys Thr Thr
                405                 410                 415

Pro Pro Val Leu Asp Ser Asp Gly Ser Phe Phe Leu Tyr Ser Lys Leu
            420                 425                 430

Thr Val Asp Lys Ser Arg Trp Gln Gln Gly Asn Val Phe Ser Cys Ser
        435                 440                 445

Val Met His Glu Ala Leu His Asn His Tyr Thr Gln Lys Ser Leu Ser
    450                 455                 460

Leu Ser Pro Gly Lys
465

<210>   44
<211>   219
<212>   PRT
<213>   Artificial Sequence

<220>
<223>   Antibody 4 light chain variable region

<400>   44
Gly Glu Leu Val Leu Thr Gln Pro Pro Ser Val Ser Glu Ala Pro Arg
1               5                   10                  15

Gln Arg Val Thr Ile Ser Cys Ser Gly Ser Ser Ser Asn Ile Gly Asn
            20                  25                  30

Asn Ala Val Asn Trp Tyr Leu Gln Val Pro Gly Thr Ala Pro Lys Leu
        35                  40                  45

Leu Ile Tyr Gly Asp His Gln Arg Pro Ser Gly Val Pro Asp Arg Phe
    50                  55                  60

Ser Gly Ser Lys Ser Gly Thr Ser Ala Ser Leu Ser Ile Ser Gly Leu
65                  70                  75                  80

Gln Ser Glu Asp Glu Ala Asp Tyr Tyr Cys Ala Ala Trp Asp Asp Ser
                85                  90                  95

Leu Asn Gly Val Val Phe Gly Gly Gly Thr Lys Leu Thr Val Leu Gly
            100                 105                 110

Arg Thr Val Ala Ala Pro Ser Val Phe Ile Phe Pro Pro Ser Asp Glu
        115                 120                 125

Gln Leu Lys Ser Gly Thr Ala Ser Val Val Cys Leu Leu Asn Asn Phe
    130                 135                 140

Tyr Pro Arg Glu Ala Lys Val Gln Trp Lys Val Asp Asn Ala Leu Gln
145                 150                 155                 160

Ser Gly Asn Ser Gln Glu Ser Val Thr Glu Gln Asp Ser Lys Asp Ser
                165                 170                 175

Thr Tyr Ser Leu Ser Ser Thr Leu Thr Leu Ser Lys Ala Asp Tyr Glu
            180                 185                 190

Lys His Lys Val Tyr Ala Cys Glu Val Thr His Gln Gly Leu Ser Ser
        195                 200                 205

Pro Val Thr Lys Ser Phe Asn Arg Gly Glu Cys
    210                 215


<210>   45
<211>   464
<212>   PRT
<213>   Artificial Sequence

<220>
<223>   Antibody 5 heavy chain variable region


<400>   45
Glu Val Gln Leu Val Gln Ser Gly Ala Glu Val Lys Lys Pro Gly Ser
1               5                   10                  15

Ser Val Lys Val Ser Cys Lys Ala Ser Gly Gly Thr Phe Ser Ser Tyr
            20                  25                  30

Ala Ile Ser Trp Val Arg Gln Ala Pro Gly Gln Gly Leu Glu Trp Met
        35                  40                  45

Gly Arg Ile Ile Pro Ile Phe Gly Ile Ala Lys Tyr Ala Gln Lys Phe
    50                  55                  60

Gln Gly Arg Val Thr Ile Thr Ala Asp Arg Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Val Thr Arg Ser Gly Asp Tyr Ser Gly Ser Ser Gly Leu Ala Phe Asp
            100                 105                 110

Ile Trp Gly Gln Gly Thr Met Val Thr Val Ser Ser Ala Ser Thr Lys
        115                 120                 125

Gly Pro Ser Val Thr Ser Ala Ser Thr Lys Gly Pro Ser Val Phe Pro
    130                 135                 140

Leu Ala Pro Ser Ser Lys Ser Thr Ser Gly Gly Thr Ala Ala Leu Gly
145                 150                 155                 160

Cys Leu Val Lys Asp Tyr Phe Pro Glu Pro Val Thr Val Ser Trp Asn
                165                 170                 175

Ser Gly Ala Leu Thr Ser Gly Val His Thr Phe Pro Ala Val Leu Gln
            180                 185                 190

Ser Ser Gly Leu Tyr Ser Leu Ser Ser Val Val Thr Val Pro Ser Ser
        195                 200                 205

Ser Leu Gly Thr Gln Thr Tyr Ile Cys Asn Val Asn His Lys Pro Ser
    210                 215                 220

Asn Thr Lys Val Asp Lys Lys Val Glu Pro Lys Ser Cys Asp Lys Thr
225                 230                 235                 240

His Thr Cys Pro Pro Cys Pro Ala Pro Glu Leu Leu Gly Gly Pro Ser
                245                 250                 255

Val Phe Leu Phe Pro Pro Lys Pro Lys Asp Thr Leu Met Ile Ser Arg
            260                 265                 270

Thr Pro Glu Val Thr Cys Val Val Val Asp Val Ser His Glu Asp Pro
        275                 280                 285

Glu Val Lys Phe Asn Trp Tyr Val Asp Gly Val Glu Val His Asn Ala
    290                 295                 300

Lys Thr Lys Pro Arg Glu Glu Gln Tyr Asn Ser Thr Tyr Arg Val Val
305                 310                 315                 320

Ser Val Leu Thr Val Leu His Gln Asp Trp Leu Asn Gly Lys Glu Tyr
                325                 330                 335

Lys Cys Lys Val Ser Asn Lys Ala Leu Pro Ala Pro Ile Glu Lys Thr
            340                 345                 350

Ile Ser Lys Ala Lys Gly Gln Pro Arg Glu Pro Gln Val Tyr Thr Leu
        355                 360                 365

Pro Pro Ser Arg Glu Glu Met Thr Lys Asn Gln Val Ser Leu Thr Cys
    370                 375                 380

Leu Val Lys Gly Phe Tyr Pro Ser Asp Ile Ala Val Glu Trp Glu Ser
385                 390                 395                 400

Asn Gly Gln Pro Glu Asn Asn Tyr Lys Thr Thr Pro Pro Val Leu Asp
                405                 410                 415

Ser Asp Gly Ser Phe Phe Leu Tyr Ser Lys Leu Thr Val Asp Lys Ser
            420                 425                 430

Arg Trp Gln Gln Gly Asn Val Phe Ser Cys Ser Val Met His Glu Ala
        435                 440                 445

Leu His Asn His Tyr Thr Gln Lys Ser Leu Ser Leu Ser Pro Gly Lys
    450                 455                 460

<210> 46
<211> 216
<212> PRT
<213> Artificial Sequence

<220>
<223> Antibody 5 light chain variable region

<400> 46
Gly Glu Leu Val Leu Thr Gln Ser Pro Gly Thr Leu Ser Leu Ser Pro
1               5                   10                  15

Gly Glu Arg Ala Thr Leu Ser Cys Arg Ala Ser Gln Ser Val Ser Ser
            20                  25                  30

Ser Tyr Leu Ala Trp Tyr Gln Gln Lys Pro Gly Gln Ala Pro Arg Leu
        35                  40                  45

Leu Ile Tyr Gly Ala Ser Ser Arg Ala Thr Gly Ile Pro Asp Arg Phe
    50                  55                  60

Ser Gly Ser Gly Ser Gly Thr Asp Phe Thr Leu Thr Ile Ser Arg Leu
65                  70                  75                  80

Glu Pro Glu Asp Phe Ala Val Tyr Tyr Cys Gln Gln Tyr Gly Ser Ser
                85                  90                  95

Pro Tyr Thr Phe Gly Gln Gly Thr Lys Val Glu Ile Lys Arg Thr Val
            100                 105                 110

Ala Ala Pro Ser Val Phe Ile Phe Pro Pro Ser Asp Glu Gln Leu Lys
        115                 120                 125

Ser Gly Thr Ala Ser Val Val Cys Leu Leu Asn Asn Phe Tyr Pro Arg
    130                 135                 140

Glu Ala Lys Val Gln Trp Lys Val Asp Asn Ala Leu Gln Ser Gly Asn
145                 150                 155                 160

Ser Gln Glu Ser Val Thr Glu Gln Asp Ser Lys Asp Ser Thr Tyr Ser
                165                 170                 175

Leu Ser Ser Thr Leu Thr Leu Ser Lys Ala Asp Tyr Glu Lys His Lys
            180                 185                 190

Val Tyr Ala Cys Glu Val Thr His Gln Gly Leu Ser Ser Pro Val Thr
        195                 200                 205

Lys Ser Phe Asn Arg Gly Glu Cys
    210                 215

<210> 47
<211> 461
<212> PRT
<213> Artificial Sequence

<220>
<223>    Antibody 6 heavy chain variable region


<400>    47
Glu Val Gln Leu Val Glu Ser Gly Gly Gly Leu Val Gln Pro Gly Arg
1               5                   10                  15

Ser Leu Arg Leu Ser Cys Ala Ala Ser Gly Phe Thr Phe Asp Asp Tyr
            20                  25                  30

Ala Met His Trp Val Arg Leu Ala Pro Gly Lys Gly Leu Glu Trp Val
        35                  40                  45

Ser Gly Ile Ser Trp Asn Ser Gly Thr Ile Gly Tyr Ala Asp Ser Val
    50                  55                  60

Lys Gly Arg Phe Thr Ile Ser Arg Asp Asn Ala Lys Asn Ser Leu Tyr
65                  70                  75                  80

Leu Gln Met Asn Ser Leu Arg Ala Glu Asp Thr Ala Leu Tyr Tyr Cys
                85                  90                  95

Ala Lys Asp Trp Ser Ser Gly Trp Ser Ser Pro Leu Asp Tyr Trp Gly
            100                 105                 110

Gln Gly Thr Leu Val Thr Val Ser Ser Ala Ser Thr Lys Gly Pro Ser
        115                 120                 125

Val Thr Ser Ala Ser Thr Lys Gly Pro Ser Val Phe Pro Leu Ala Pro
    130                 135                 140

Ser Ser Lys Ser Thr Ser Gly Gly Thr Ala Ala Leu Gly Cys Leu Val
145                 150                 155                 160

Lys Asp Tyr Phe Pro Glu Pro Val Thr Val Ser Trp Asn Ser Gly Ala
                165                 170                 175

Leu Thr Ser Gly Val His Thr Phe Pro Ala Val Leu Gln Ser Ser Gly
            180                 185                 190

Leu Tyr Ser Leu Ser Ser Val Val Thr Val Pro Ser Ser Ser Leu Gly
        195                 200                 205

Thr Gln Thr Tyr Ile Cys Asn Val Asn His Lys Pro Ser Asn Thr Lys
    210                 215                 220

Val Asp Lys Lys Val Glu Pro Lys Ser Cys Asp Lys Thr His Thr Cys
225                 230                 235                 240

Pro Pro Cys Pro Ala Pro Glu Leu Leu Gly Gly Pro Ser Val Phe Leu
                245                 250                 255

Phe Pro Pro Lys Pro Lys Asp Thr Leu Met Ile Ser Arg Thr Pro Glu
            260                 265                 270

Val Thr Cys Val Val Val Asp Val Ser His Glu Asp Pro Glu Val Lys
        275                 280                 285

Phe Asn Trp Tyr Val Asp Gly Val Glu Val His Asn Ala Lys Thr Lys
    290                 295                 300

Pro Arg Glu Glu Gln Tyr Asn Ser Thr Tyr Arg Val Val Ser Val Leu
305                 310                 315                 320

Thr Val Leu His Gln Asp Trp Leu Asn Gly Lys Glu Tyr Lys Cys Lys
                325                 330                 335

Val Ser Asn Lys Ala Leu Pro Ala Pro Ile Glu Lys Thr Ile Ser Lys
            340                 345                 350

Ala Lys Gly Gln Pro Arg Glu Pro Gln Val Tyr Thr Leu Pro Pro Ser
        355                 360                 365

Arg Glu Glu Met Thr Lys Asn Gln Val Ser Leu Thr Cys Leu Val Lys
    370                 375                 380

Gly Phe Tyr Pro Ser Asp Ile Ala Val Glu Trp Glu Ser Asn Gly Gln
385                 390                 395                 400

Pro Glu Asn Asn Tyr Lys Thr Thr Pro Pro Val Leu Asp Ser Asp Gly
                405                 410                 415

Ser Phe Phe Leu Tyr Ser Lys Leu Thr Val Asp Lys Ser Arg Trp Gln
            420                 425                 430

Gln Gly Asn Val Phe Ser Cys Ser Val Met His Glu Ala Leu His Asn
        435                 440                 445

His Tyr Thr Gln Lys Ser Leu Ser Leu Ser Pro Gly Lys
    450                 455                 460

<210>    48
<211>    11
<212>    PRT
<213>    Artificial Sequence

<220>
<223>    Antibody 6 light chain variable region

<400>    48
Gln Ser Tyr Asp Ser Ser Leu Ser Gly Val Val
1               5                   10